# EUROPÄISCHE PATENTSCHRIFT

(11) **EP 2 884 946 B1**
(45) Veröffentlichungstag und Bekanntmachung des Hinweises auf die Patenterteilung: **02.01.2019**
(21) Anmeldenummer: 13745106.8
(22) Anmeldetag: 05.08.2013
(51) Int. Cl.: A61F 2/24, A61F 2/90, A61B 17/00, A61F 2/856

(54) **IMPLANTIERBARE EINRICHTUNG ZUR VERWENDUNG IM MENSCHLICHEN UND/ODER TIERISCHEN KÖRPER ZUM ERSATZ EINER ORGANKLAPPE**
IMPLANTABLE DEVICE FOR USE IN THE HUMAN AND/OR ANIMAL BODY TO REPLACE AN ORGAN VALVE
DISPOSITIF IMPLANTABLE DESTINÉ À ÊTRE UTILISÉ DANS UN CORPS HUMAIN ET/OU ANIMAL POUR REMPLACER UN CLAPET ORGANIQUE

(30) Priorität: 15.08.2012 DE 102012107465
(43) Veröffentlichungstag der Anmeldung: 24.06.2015
(73) Patentinhaber: pfm medical ag, 50966 Köln (DE)
(72) Erfinder: FREUDENTHAL, Franz, La Paz 23000 (BO)
(74) Vertreter: Hohendorf, Carsten
(86) Internationale Anmeldenummer: PCT/EP2013/066385
(87) Internationale Veröffentlichungsnummer: WO 2014/026870

(56) Entgegenhaltungen:
- EP-A1- 1 849 440
- WO-A1-2008/100599
- WO-A2-2011/072084
- WO-A2-2011/143263
- FR-A1- 2 874 813
- US-A1- 2005 137 686
- US-A1- 2011 160 836

## Beschreibung

Die Erfindung betrifft eine implantierbare Einrichtung zur Verwendung im menschlichen und/oder tierischen Körper zum Ersatz einer Organklappe umfassend einen Grundkörper mit einem ersten Ende und einem zweiten Ende, wobei das erste Ende und das zweite Ende jeweils eine Öffnung aufweisen, um eine Fluidverbindung zwischen dem ersten Ende und dem zweiten Ende durch den Grundkörper bereitzustellen; ein erstes Membranelement angeordnet innerhalb oder an einem Ende des Grundkörpers, wobei das Membranelement derart ausgebildet ist, dass es die Fluidverbindung durch den Grundkörper in eine erste Flussrichtung freigibt und in eine zweite Flussrichtung (entgegengesetzt zu der ersten Flussrichtung) sperrt; wobei der Grundkörper in einem ersten Betriebszustand (Primärform) ein großes Verhältnis von Länge zu Querausdehnung entlang der Längsachse des Grundkörpers und in einem zweiten Betriebszustand (Sekundärform) ein kleineres Verhältnis von Länge zu Querausdehnung entlang der Längsachse des Grundkörpers aufweist; und wobei der Grundkörper durch Aufbringen einer Kraft gegen elastische Materialkräfte aus der Sekundärform in die Primärform reversibel überführbar ist.

Derartige implantierbare Einrichtungen zum Ersatz einer Organklappe im menschlichen und/oder tierischen Körper sind im Stand der Technik bekannt. Es war früher üblich, insbesondere Herzklappen chirurgisch am offenen Herzen zu ersetzen, was eine nicht ungefährliche Operation darstellt, insbesondere bei älteren Patienten. Es wurden daher Einrichtungen zum Herzklappenersatz entwickelt, die über einen Katheter ohne eine Operation am offenen Herzen an die entsprechende Position im Herzen gebracht werden können. Beispielsweise ist es aus der EP 0 592 410 bekannt, eine zusammendrückbare elastische Klappe, die auf einem elastischen Stent angebracht ist, vorzusehen, wobei die Kommissurpunkte der elastischen zusammendrückbaren Klappe auf der Zylinderoberfläche des elastischen Stents angebracht sind. Die elastische zusammendrückbare Klappe ist eine biologische, dreilappige Klappe. Der Stent besteht aus einem Edelstahldraht, der in einer Anzahl von Schleifen gefaltet und kreisförmig zusammengebogen und zusammengeschweißt ist. Der Stent enthält zwei oder mehr geschlossene Ringe, die miteinander verbunden sind, um eine zylindrische Struktur zu bilden. Drei der Schleifen im äußeren Ring sind mit einer größeren Höhe ausgebildet als die anderen Schleifen, um Spitzen zu bilden, an denen die Kommissurpunkte der biologischen Klappe angebracht sind. Die zylindrische Oberfläche des Stents kann auch geschlossen ausgebildet sein. Aufgrund der Rohr- bzw. Ringform des Stents ist eine nur verhältnismäßig schlechte Verankerung im Implantationsbereich, insbesondere in der Aorta und im Herzen möglich.

Eine weiterentwickelte Verankerung für einen Herzklappenersatz wird in der DE 101 21 210 A1 beschrieben. Gemäß dieser Druckschrift ist ein intraluminares Verankerungselement von der Zylinderform abweichend so geformt, dass es in Gebrauchsstellung zumindest bereichsweise mit der Aorta formschlüssig verbunden ist. Das intraluminare Verankerungselement gemäß dieser Druckschrift weist daher sich in radialer Richtung erstreckende Erweiterungen am Herzausgang (hinter der ursprünglichen Aortenklappe) auf. Außerdem ist es dem gekrümmten Verlauf der Aorta angepasst gekrümmt. Das Verankerungselement ist außerdem aus einer beispielsweise gitterförmigen, schleifenförmigen oder schraubenförmigen Fadenstruktur bzw. Filamenten aufgebaut und kann mehrere meanderförmige, ringbildende Fadenstrukturen umfassen. Die einzelnen ringbildenden Strukturen sind untereinander bzw. miteinander durch Kleben, Löten, Verschweißen etc. verbunden. Bei dieser Ausführungsform eines Herzklappenersatzes ergibt sich der Nachteil, dass das Verankerungselement sehr lang ausgeführt ist, also sehr tief in ein Blutgefäß bzw. das Herz eingebracht werden muss. Zwar sind Öffnungen für die Abgänge von verschiedenen Koronararterien vorgesehen, jedoch kann es aufgrund der Länge des Verankerungselementes dazukommen, dass diese teilweise von dem Verankerungselement abgedeckt werden, so dass es dort zu einer Blockierung des Blutflusses bzw. Verstopfen der Abgänge kommen kann.

Aus der EP 1 057 460 A1 bzw. dem Abstract der JP 2001/000460 A ist es bekannt, eine Herzkappenersatzeinrichtung, die einen Stent aufweist, vorzusehen, wobei der Stent in radialer Richtung des Blutgefäßes ausdehnbar ist und eine biologische Klappe an dem Stent befestigt ist. Die Stentklappenanordnung wird auf den expandierenden Teil eines Ballonkatheters aufgebracht und in den Körper eines Menschen eingeführt. Der Stent ist aus einer Vielzahl von Abschnitten zusammengesetzt, die aus Draht gebildet sind. Die einzelnen Drahtabschnitte sind zusammengeschweißt. Mittels des Ballonkatheters wird der Stent am Implantationsort auf den gewünschten Durchmesser expandiert, was in zwei Stufen geschieht. Nach der Expansion des Stents am Implantationsort wird der Durchmesser des Ballonkatheters wieder verringert und der Katheter entfernt. Die Pulmonalklappenersatzeinrichtung verbleibt in der Pulmonalarterie, die Arterienwand berührend. Als nachteilig erweist sich bei dieser Herzklappenersatzeinrichtung, dass ein Ballonkatheter zum Expandieren des Stents verwendet werden muss. Darüber hinaus soll der Stent lediglich aufgrund seiner expandierten Form in dem Gefäß bzw. der Arterie halten. Es hat sich jedoch gezeigt, dass bei dieser Form eines Herzklappenersatzes Probleme durch ein Verschieben des Stents innerhalb des Gefäßes auftreten können, insbesondere da bei nicht exakter Positionierung die Abgänge der Koronargefäße blockiert werden können, so dass es zu einem zumindest teilweisen Verschließen dieser Abgänge und damit zu einem stockenden Blutfluss kommen kann. Außerdem treten Probleme auf, sofern eine Fehlpositionierung erfolgt ist, da mittels des Ballonkatheters zwar der Stent expandiert, nicht jedoch in seinem Durchmesser wieder reduziert werden kann.

Aus der US 5,855,597 ist es außerdem bekannt, sternförmige Elemente auszuschneiden und diese zu einem Stent zusammenzufügen. Ein Aortenklappenersatz aus einem flexiblen, biokompatiblen Material wird in eine zentrale Öffnung der aneinander gefügten sternförmigen Elemente eingefügt. Über ein Kathetersystem wird der Stent an den gewünschten Implantationsort gebracht. Zwar wird durch die Sternform ein Halt innerhalb der Aorta des Patienten geschaffen, jedoch besteht dort auch Verletzungsgefahr, insbesondere wenn das Blutgefäß aufgrund des Alters oder sonstigen Gesundheitszustand des Patienten leicht verletzlich, insbesondere perforierbar ist.

Aus der US 6,482,228 B1 ist beispielsweise ein Aortenklappenersatz bekannt, der einen Stent und davon abgesetzt, jedoch damit verbunden, einen rotorförmigen Klappenersatz aufweist. Dieser wird über der ursprünglichen Klappe angeordnet. Der Stent besteht aus mehreren aneinander gefügten Ringen von wellenlinienförmig gebogenen Drähten. Als nachteilig erweist sich hier der Aufbau des Aortenklappenersatzes durch Vorsehen eines Stents mit davon abgesetztem rotorförmigen Element zur Anordnung hinter der natürlichen Aortenklappe. Dies ist zum einen sehr aufwendig und zum anderen besteht die Gefahr, dass der Rotor sich von dem Stent löst. Außerdem ist er im Wesentlichen ohne weiteren Halt durch den Stent - lediglich in Längsrichtung gesichert - innerhalb der Aorta angeordnet. Der Aortenklappenersatz stellt somit keine feste und stabile Einheit dar.

Zum Herzklappenersatz sind im Stand der Technik außerdem ringförmige Einrichtungen bekannt, die an drei Stellen aus dem Ring herausgezogene Pfostenelemente aufweisen. Diese können entweder schlaufenförmig sein, wie dies in der WO 97/46177 offenbart ist, oder aus einem Vollmaterial bestehen, wie beispielsweise in der US 4,816,029, DE 196 24 948 A1 und der DE 35 41 478 A1 offenbart. Diese ringförmigen Herzklappenersatzeinrichtungen sind jedoch alle nicht über einen Katheter implantierbar, da sie sich nicht auf eine entsprechend geringe Größe zusammenlegen lassen.

Aus der DE 103 34 868 ist weiterhin eine implantierbare Einrichtung zur Verwendung im menschlichen und/oder tierischen Körper zum Ersatz einer Organklappe bekannt. Die offenbarte implantierbare Einrichtung besteht aus einem Grundkörper mit einem ersten und einem zweiten mit Öffnungen versehenen Ende und mit einem mit zumindest einer Öffnung versehenen Membranelement. Die Einrichtung hat in einem ersten Betriebszustand ein großes Verhältnis von Länge zu Querausdehnung entlang einer Achse und in einem zweiten Betriebszustand ein kleineres Verhältnis von Länge zur Querausdehnung entlang der Achse, wobei die Einrichtung durch Aufbringen einer Kraft gegen elastische Materialkräfte aus der Sekundärform in die Primärform reversibel überführbar ist. Weiterhin weist die implantierbare Vorrichtung zumindest an einem der beiden Enden des Grundkörpers einen auskragenden Verankerungsabschnitt zum Verankern der Einrichtung in einem Organ und/oder einem Gefäß auf. Der Grundkörper, der implantierbaren Einrichtung gemäß der DE 103 34 868 A1 ist einstückig ausgebildet und beispielsweise aus einem Materialstück geschnitten und/oder gestanzt und/oder durch ein anderes Trennverfahren herausgetrennt. Daraus ergibt sich der Nachteil, dass die implantierbare Einrichtung eine relativ hohe Steifigkeit aufweist und beispielsweise zum Erzeugen unterschiedlicher Steifigkeiten in zumindest einem Teilbereich des Grundkörpers chemisch und/oder mechanisch behandelt, insbesondere geätzt, elektropoliert, mikrogeschliffen oder anderweitig behandelt werden muss. Weiterhin besteht die Gefahr bei einer zu starken Nachbehandlung des Grundkörpers, dass einzelnen Elemente des Grundkörpers, insbesondere die daraus geformten Streben, bei einem Verformen der implantierbaren Einrichtung von der Primärform in die Sekundärform beschädigt werden.

Die WO 2011/143263 A2 offenbart eine Stützstruktur für eine Herzklappenprothese, umfassend mindestens einen Strang, der so geflochten ist, dass er eine röhrenförmige Struktur bildet, die eine erste Konfiguration und eine zweite Konfiguration aufweist. Die röhrenförmige Struktur umfasst in der ersten Konfiguration ein erstes Ende und ein zweites Ende und einen länglichen Körper zwischen dem ersten Ende und dem zweiten Ende. Der geflochtene Strang weist eine Formgedächtniseigenschaft auf und die röhrenförmige Struktur ist in der zweiten Konfiguration auf eine gefaltete Form voreingestellt, die mindestens eine Falte umfasst, so dass der längliche Körper so angepasst ist, dass er allmählich solch eine gefaltete zweite Konfiguration, bei welcher es sich um die Anwendungskonfiguration handelt, wiedererlangt, wenn er bei der Anwendung aus einem Führungskatheter geschoben wird. Dadurch umfasst die röhrenförmige Struktur in der zweiten Konfiguration die mindestens eine Falte, welche die röhrenförmige Struktur in Längsrichtung verkürzt und einen Abschnitt des Körpers erzeugt, der mindestens doppelt so viele Schichten aufweist wie die röhrenförmige Struktur in der ersten Konfiguration.

Der vorliegenden Erfindung liegt die Aufgabe zugrunde, eine implantierbare Einrichtung zum Ersatz einer Organklappe bereitzustellen, welche eine hohe Flexibilität aufweist, ohne die Gefahr, dass einzelnen Elemente der implantierbaren Einrichtung bei einem Verformen beschädigt werden. Insbesondere liegt der Erfindung die Aufgabe zugrunde, eine implantierbare Einrichtung bereitzustellen, die in unterschiedlichen Bereichen unterschiedliche Steifigkeiten aufweist.

Die Aufgabe wird gelöst durch eine implantierbare Einrichtung zur Verwendung im menschlichen und/oder tierischen Körper zum Ersatz einer Organklappe umfassend einen Grundkörper mit einem ersten Ende und einem zweiten Ende, wobei das erste Ende und das zweite Ende jeweils eine Öffnung aufweisen um eine Fluidverbindung zwischen dem ersten Ende und dem zweiten Ende durch den Grundkörper bereitzustellen; und ein erstes Membranelement angeordnet innerhalb oder an einem Ende des Grundkörpers, wobei das Membranelement derart ausgebildet ist, dass es die Fluidverbindung durch den Grundkörper in eine erste Flussrichtung freigibt und in eine zweite Flussrichtung, entgegengesetzt zu der ersten Flussrichtung, sperrt; wobei der Grundkörper in einem ersten Betriebszustand (Primärform) ein großes Verhältnis von Länge zu Querausdehnung entlang der Längsachse des Grundkörpers und in einem zweiten Betriebszustand (Sekundärform) ein kleineres Verhältnis von Länge zu Querausdehnung entlang der Längsachse des Grundkörpers aufweist; und wobei der Grundkörper durch Aufbringen einer Kraft gegen elastische Materialkräfte aus der Sekundärform in die Primärform reversibel überführbar ist; und wobei der Grundkörper aus einem einzigen drahtartigen Element oder mehreren miteinander verbundenen drahtartigen Elementen durch verschränkendes Wickeln und/oder Verwinden und/oder Verflechten nach Art eines Gewebes und/oder Geleges und/oder Netzes geformt ist.

Durch die Ausbildung des Grundkörpers aus einem einzigen drahtartigen Element oder mehreren miteinander verbundenen drahtartigen Elementen durch verschränkendes Wickeln und/oder Verwinden und/oder Verflechten nach Art eines Gewebes und/oder Geleges und/oder Netzes wird eine implantierbare Einrichtung geschaffen, die eine hohe Flexibilität aufweist, bei einem gleichzeitigen sicheren Sitz an der Implantationsstelle. In der Sekundärform legt sich die implantierbare Einrichtung beispielsweise an eine Gefäßwand an. Aufgrund der elastischen Materialkräfte wirkt eine Kraft in radialer Richtung, welche die implantierbare Einrichtung an der Implantationsstelle fixiert. Da der Grundkörper reversibel von einer Primärform in eine Sekundärform überführbar ist, kann er problemlos über einen Katheter an den Implantationsort gebracht werden. Beim Herausschieben aus dem Katheter entfaltet sich die implantierbare Einrichtung aus der Primärform in die Sekundärform, wobei sich der Durchmesser des Grundkörpers vergrößert und dafür im Allgemeinen die Länge verringert. Durch die Möglichkeit des reversiblen Überführens der Primär- in die Sekundärform und umgekehrt, der Sekundär- in die Primärform, kann im Gegensatz zu dem Stent nach der EP 1 057 460 A1 auch ein Zurückziehen der Einrichtung in den Katheter erfolgen, sofern während der Implantation festgestellt wird, dass diese nicht ordnungsgemäß verläuft, also insbesondere die implantierbare Einrichtung nicht ordnungsgemäß zu den Abgängen der Koronargefäße und/oder der natürlichen Herzklappe und/oder der Aorta sowie der Herzkammer positioniert ist. Aufgrund der Verwendung eines einzigen drahtartigen Elements oder von mehreren miteinander verbundenen drahtartigen Elementen, welches durch verschränkendes Wickeln und/oder Verwinden und/oder Verflechten nach Art eines Gewebes und/oder Geleges und/oder Netzes die implantierbare Einrichtung ausformt, ergibt sich der Vorteil, dass die erfindungsgemäße implantierbare Einrichtung eine hohe Flexibilität aufweist, ohne dass die Gefahr besteht, dass einzelne Elemente der implantierbaren Einrichtung bei der Verformung von der Primärform in die Sekundärform bzw. von der Sekundärform in die Primärform brechen. Die Ausbildung der implantierbaren Einrichtung, insbesondere des Grundkörpers, aus einem einzigen drahtartigen Element hat weiterhin den Vorteil, dass keine Verbindungspunkte, beispielsweise Schweißpunkte, zwischen einzelnen Elementen des Grundkörpers vorhanden sind, die besonders leicht brechen können. Gerade ein solches Auseinanderbrechen von einzelnen Elementen einer implantierbaren Einrichtung kann dazu führen, dass scharfkantige Bereiche aus einer Vorrichtung herausragen, die die Wand, insbesondere die Aorta verletzen bzw. perforieren können. Die Struktur des Grundkörpers kann außerdem bei einstückiger Ausbildung gleichmäßiger gestaltet werden als dies beim Zusammenfügen von einzelnen ringförmigen Elementen, wie im Stand der Technik beschrieben, möglich ist. Die Ausbildung der implantierbaren Einrichtung aus mehreren miteinander verbundenen drahtartigen Elementen hat den Vorteil, dass die implantierbare Einrichtung auf einfache Art und Weise maschinell hergestellt werden kann.

Gemäß einer Variante der Erfindung weist der Grundkörper in dem zweiten Betriebszustand (Sekundärform) an dem ersten Ende und/oder dem zweiten Ende wenigstens einen sich in radialer Richtung des Grundkörpers erstreckenden Verankerungsabschnitt zum Verankern der Einrichtung in einem Organ und/oder Gefäß auf. Dadurch wird eine implantierbare Einrichtung zum Ersatz einer Organklappe geschaffen, mittels derer aufgrund der Verwendung von wenigstens einem auskragenden Verankerungsabschnitt an einem Ende des Grundkörpers ein besonders guter Halt in einem Gefäß und/oder Organ möglich ist. Bei der Verwendung der implantierbaren Einrichtung als Herzklappenersatz kann das eine Ende mit dem auskragenden Verankerungsabschnitt beispielsweise in die linke Herzkammer hineinragen und sich dort festhalten und das andere Ende des Grundkörpers an die Aortenwand anlegen. Dadurch kann die implantierbare Einrichtung bzw. der Grundkörper viel kürzer ausgebildet werden, als dies beispielsweise bei dem Stent nach der DE 101 21 210 A1 möglich ist.

Zweckmäßigerweise ist der sich in dem zweiten Betriebszustand (Sekundärform) an dem ersten Ende und/oder dem zweiten Ende angeordnete wenigstens eine sich in radialer Richtung des Grundkörpers erstreckende Verankerungsabschnitt um den Umfang des Grundkörpers am ersten Ende und/oder zweiten Ende des Grundkörpers umlaufend vorgesehen. Alternativ können jedoch auch einzelne auskragende Verankerungsabschnitte am Umfang des Grundkörpers vorgesehen werden. Je nach Implantationsort kann die eine oder andere Variante bevorzugt werden, wobei die Auswahl insbesondere von den Platzverhältnissen und eventuellen Verengungen in einem Gefäß durch Kalkablagerungen etc. abhängig gemacht werden kann.

In einer Variante der Erfindung weist die implantierbare Einrichtung Bereiche unterschiedlicher Steifigkeit auf. Diese Bereiche unterschiedlicher Steifigkeit können beispielsweise durch unterschiedliches verschränkendes Wickeln und/oder Verwinden und/oder Verflechten des einzigen drahtartigen Elements erzeugt werden oder durch ein drahtartiges Element mit unterschiedlichen Querschnitten, insbesondere rund, oval und/oder mehreckig. Besonders bevorzugt ist ein Bereich geringerer Steifigkeit zwischen Bereichen größerer Steifigkeit angeordnet. Besonders bevorzugt ist der Bereich geringerer Steifigkeit im Grundkörper außerhalb des wenigstens einen Verankerungsabschnitts vorgesehen. Durch das Vorsehen von Bereichen unterschiedlicher Steifigkeit können Biegungen am Implantationsort nachgebildet werden, wie beispielsweise die Biegungen einer Aorta. Um einen guten Halt am Implantationsort sicherzustellen weist der Bereich des wenigstens einen Verankerungsabschnitts, also das erste und/oder zweite Ende des Grundkörpers, vorzugsweise eine größere Steifigkeit auf als ein oder mehrere dazwischen angeordnete Abschnitte. Es können auch mehrere Bereiche unterschiedlicher Steifigkeit über die Länge des Grundkörpers hinweg vorgesehen werden, wenn die äußeren Gegebenheiten, insbesondere in der Aorta oder im Herzkammerbereich, dies erfordern. Die Verteilung der Steifigkeiten über den Grundkörper hinweg kann somit auch an den jeweiligen Patienten und die in dessen Herzen bzw. am Implantationsort herrschenden räumlichen Gegebenheiten angepasst werden.

Grundsätzlich ist es möglich, die implantierbare Einrichtung gemäß der vorliegenden Erfindung auf den jeweiligen Patienten abgestimmt auszuführen und/oder eine Standardform vorzusehen, die im Wesentlichen für den größten Teil der Patienten verwendbar ist. Insbesondere können an einer Grundform auch bereits variabel veränderbare Bereiche vorgesehen werden, die eine Anpassung wiederrum in den größten Teil der Ausnahmefälle erlaubt. Dadurch werden die Kosten für die Herstellung der implantierbaren Einrichtung ebenfalls reduziert, da wirkliche Sonderanfertigungen dadurch recht selten werden.

In einer Variante der erfindungsgemäßen implantierbaren Einrichtung weist der Grundkörper in dessen Umfangswandung wenigstens eine Öffnung auf, um eine Fluidverbindung zwischen dem Inneren des Grundkörpers und einem Gefäß des menschlichen oder tierischen Körpers bereitzustellen. Am Implantationsort ist die erfindungsgemäße implantierbare Einrichtung in der Sekundärform beispielsweise derart angeordnet, dass der wenigstens eine Verankerungsabschnitt in eine Herzkammer hineinragt, wo er sich festhält und der verbleibende Teil des Grundkörpers, welcher im Wesentlichen zylinderförmig ist, in ein Gefäß hineinragt, wie beispielsweise die Aorta, wobei der verbleibende Teil des Grundkörpers sich an die Aortenwand anlegt. Durch die wenigstens eine Öffnung in der Umfangswandung des Grundkörpers kann eine Fluidverbindung zu einem beispielsweise von der Aorta abgehenden Gefäß des menschlichen oder tierischen Körpers bereitgestellt werden, wie beispielsweise einer Koronararterie. Zweckmäßigerweise weist die wenigstens eine Öffnung einen einer Koronararterie entsprechenden Durchmesser auf, so dass ein Blutfluss in diesem Bereich nicht behindert wird.

In einer besonders zweckmäßigen Variante der erfindungsgemäßen implantierbaren Einrichtung weist der Grundkörper in dessen Umfangswandung zwei Öffnungen auf, welche im implantierten Zustand der erfindungsgemäßen Einrichtung derart angeordnet sind, dass sich die zwei Öffnungen mit den Koronararterien überdecken.

Nach einer weiteren Variante der Erfindung ist die wenigstens eine Öffnung in der Umfangswandung des Grundkörpers außerhalb des wenigstens einen Verankerungsabschnitts vorgesehen, so dass die Funktion des Verankerungsabschnitts gewährleistet bleibt. Die implantierbare Einrichtung gemäß der Erfindung ist also zweckmäßigerweise derart in dem menschlichen oder tierischen Körper angeordnet, dass der Verankerungsabschnitt keine abgehenden Gefäße des menschlichen oder tierischen Körpers verschließt.

In einer besonders vorteilhaften Variante der erfindungsgemäßen implantierbaren Einrichtung wird die wenigstens eine Öffnung in der Umfangswandung des Grundkörpers durch ein weiteres Verwinden, Verflechten oder Wickeln des einzigen drahtartigen Elementes des Grundkörpers erzeugt, wobei sich durch das weitere Verwinden, Verflechten oder Wickeln des einzigen drahtartigen Elements des Grundkörpers im Bereich der wenigstens einen Öffnung in der Umfangswandung des Grundkörpers eine größere Maschenweite ergibt als im übrigen Bereich der Umfangswandung des Grundkörpers. Zweckmäßigerweise ist die größere Maschenweite derart ausgebildet, dass sich im Bereich der abgehenden Gefäße kein Element des weiter verwundenden, verflochtenen oder gewickelten Grundkörpers befindet.

Nach einer weiteren Variante der Erfindung umfasst die implantierbare Einrichtung mindestens eine röntgendichte Markierung, insbesondere in Form von Markerplaketten, Markierungen oder Markerdrähten. Die röntgendichte Markierung ist insbesondere im Bereich des Grundkörpers angeordnet. Hierdurch ist es möglich, während des Implantationsvorgangs die Positionierung, insbesondere hinsichtlich der Abgänge zu Koronargefäßen über einen Monitor oder dergleichen zu verfolgen. Hierzu eignen sich insbesondere die Röntgendarstellung oder Magnetresonanztomographie, die eine achsengenaue Position der implantierbaren Einrichtung, die insbesondere ein Aortenklappenersatz ist, im Körper des Patienten wiedergeben können. Die Markierungen können an verschiedenen Stellen des Grundkörpers, bzw. der implantierbaren Einrichtung, insbesondere in Bereichen der Öffnungen im Grundkörper, vorgesehen werden.

Zweckmäßigerweise besteht die implantierbare Einrichtung gemäß der Erfindung ganz oder teilweise aus einem Formgedächtnismaterial, insbesondere Nitinol oder einem Kunststoff mit Formgedächtnis.

Eine weitere Variante der Erfindung sieht vor, dass die implantierbare Einrichtung ganz oder teilweise aus einem resorbierbaren Material besteht.

In einer Variante der Erfindung besteht das erste Membranelement aus einem synthetischen oder biologischen Material, insbesondere aus Polyurethan.

Nach einer besonders bevorzugten Variante der Erfindung ist das erste Membranelement mit einer Beschichtung versehen, zum Schaffen einer Biostabilität. Diese Beschichtung zum Beschaffen einer Biostabilität ist vorzugsweise eine Titanbeschichtung.

Grundkörper und erstes Membranelement der implantierbaren Einrichtung sind nach einer Variante lösbar oder unlösbar miteinander verbindbar oder verbunden, insbesondere durch Kleben, Schweißen, Nähen, Aufschmelzen, Tauchen oder einem anderen Fügeverfahren.

Es erweist sich als besonders vorteilhaft, wenn das erste Membranelement für die implantierbare Einrichtung einen Ringabschnitt und einen mit diesem verbundenen Klappenaufschnitt aufweist. Vorzugsweise umfasst der Klappenabschnitt drei Segelelemente. Hierdurch kann insbesondere eine natürliche Klappe nachgebildet werden. Durch Vorsehen eines Ringabschnitts ist eine gute Verankerung an dem Grundkörper der implantierbaren Einrichtung möglich. Der Grundkörper besteht bevorzugt aus einem biokompatiblen Material, insbesondere einem Metall oder einer Metalllegierung, insbesondere einem Edelstahl oder einem Kunststoff, wie Polycarbonat, insbesondere einem Formgedächtnismaterial, wie Nitinol. Das erste Membranelement besteht bevorzugt aus einem synthetischen oder biologischen Material, insbesondere aus Polyurethan. Grundkörper und erstes Membranelement können lösbar oder unlösbar miteinander verbunden sein oder werden. Ein Verbinden von Grundkörper und erstem Membranelement kann daher besonders bevorzugt durch Kleben, Schweißen, Nähen, Aufschmelzen oder Tauchen oder ein anderes Fügeverfahren erfolgen. Der Ringabschnitt des ersten Membranelements wird vorzugsweise so breit gewählt, dass ein guter Halt an dem Grundkörper ermöglicht wird. Da das erste Membranelement im Allgemeinen aus einem sehr dünnen Material besteht, kann der Ringabschnitt beispielsweise als dünner Schlauch auf dem Grundkörper aufgebracht bzw. in diesem eingefügt werden. Hierbei eignet sich besonders ein Tauchen des Grundkörpers bzw. ein Aufbringen eines dünnen Membranabschnitts auf der Innen- und der Außenseite des Grundkörpers. Hierdurch ist auch ein Schutz gegen Verrutschen des ersten Membranelements gegenüber dem Grundkörper gegeben.

Die erfindungsgemäße implantierbare Einrichtung kann besonders in der Erwachsenenkardiologie, hierbei besonders bevorzugt bei mit zunehmendem Lebensalter häufiger auftretender Aortenklappeninsuffizienz verwendet werden. Die erfindungsgemäße implantierbare Einrichtung kann jedoch auch zum Ersatz einer Trikuspidalklappe, Pulmonalklappe oder Bikuspidalklappe (Mitralklappe) eingesetzt werden.

Das erste Membranelement der erfindungsgemäßen implantierbaren Einrichtung ist im zweiten Betriebszustand (Sekundärform) am ersten Ende des Grundkörpers, am zweiten Ende des Grundkörpers oder zwischen dem ersten und dem zweite Ende des Grundkörpers angeordnet, vorzugsweise mittig in Längsrichtung des Grundkörpers zwischen dem ersten und dem zweiten Ende des Grundkörpers. Die erfindungsgemäße implantierbare Einrichtung weist in einer Variante der Erfindung im zweiten Betriebszustand (Sekundärform) am ersten Ende und/oder zweiten Ende wenigstens ein zweites Membranelement auf, um die Öffnung am ersten und/oder zweiten Ende teilweise fluiddicht zu verschließen. Zweckmäßigerweise sind das erste Membranelement und das zweite Membranelement am ersten Ende des Grundkörpers oder am zweiten Ende des Grundkörpers benachbart zueinander angeordnet. Das zweite Membranelement dient dazu, die Verbindungsstelle zwischen dem ersten Membranelement und dem Grundkörper fluiddicht zu verschließen, so dass im Bereich der Verbindungsstelle zwischen dem ersten Membranelement und dem Grundkörper keine Flüssigkeit hindurchtreten kann.

Zweckmäßigerweise ist die implantierbare Einrichtung gemäß der Erfindung replazierbar und/oder explantierbar ausgebildet. Die Replazierbarkeit bzw.

Explantierbarkeit ergibt sich daraus, dass die implantierbare Einrichtung reversibel von der Primärform in die Sekundärform und umgekehrt von der Sekundärform in die Primärform überführbar ist. Dadurch kann die implantierbare Einrichtung gemäß der Erfindung beispielsweise während des Implantationsvorgangs in den Katheter zurückgezogen werden, falls während der Implantation festgestellt wird, dass diese nicht ordnungsgemäß verläuft, also insbesondere, dass die implantierbare Einrichtung nicht ordnungsgemäß zu den Abgängen der Koronargefäße und/oder der natürlichen Herzklappe und der Aorta sowie der Herzkammer positioniert ist.

In einer bevorzugten Variante der erfindungsgemäßen implantierbaren Einrichtung ist die Einrichtung im zweiten Betriebszustand (Sekundärform) zwischen dem ersten und dem zweiten Ende des Grundkörpers wenigstens teilweise, vorzugsweise vollständig, in radialer Richtung deformierbar ausgebildet, so dass sich der Grundkörper an eine Gefäßwandung und/oder den Rand einer Öffnung und/oder den Rand einer defekten Organklappe anlegen kann. Dadurch, dass sich der Grundkörper bei der Implantation an eine Gefäßwandung und/oder den Rand einer Öffnung und/oder den Rand einer defekten Organklappe anlegt, wird erreicht, dass sich die erfindungsgemäße implantierbare Einrichtung nach der Implantation nicht selbstständig, beispielsweise verursacht durch die Pumpbewegung des Herzens, replaziert. Weiterhin wird dadurch erreicht, dass sich die implantierbare Einrichtung selbständig in der Öffnung des menschlichen oder tierischen Körpers zentriert.

Beispielsweise wird die erfindungsgemäße implantierbare Einrichtung während der Implantation in den menschlichen und/oder tierischen Körper soweit in den Bereich einer Herzklappe, insbesondere einer Aortenklappe, eingeschoben, dass die natürliche Klappe während der Überführung der erfindungsgemäßen implantierbaren Einrichtung von dem ersten Betriebszustand (Primärform) in den zweiten Betriebszustand (Sekundärform) an die Gefäßwand gedrückt und durch die implantierbare Einrichtung dort gehalten wird. Hierbei wäre es grundsätzlich sogar möglich, in eine bereits implantierte Einrichtung eine weitere implantierbare Einrichtung einzufügen, wobei das erste Membranelement der zuerst implantierten Einrichtung dabei ebenfalls an die Wand des Grundkörpers gedrückt würde. Ein solches Einfügen einer weiteren implantierbaren Einrichtung in eine bereits implantierte Einrichtung könnte sich beispielsweise bei nachlassender Stabilität und Beweglichkeit des ersten Membranelements als sinnvoll erweisen. Auch ist es grundsätzlich möglich, nach vorherigem Entfernen einer alten natürlichen Klappe, insbesondere durch Operation, an dieser Stelle eine implantierbare Einrichtung mit Membranelement als Klappenersatz einzufügen. Gerade bei starker Verkalkung der natürlichen Herzklappe kann es sich als vorteilhaft erweisen, diese vollständig zu entfernen, da sie dann üblicherweise im Wesentlichen unbeweglich geworden ist. In diesem Fall wäre es ansonsten relativ schwer möglich, die natürliche Klappe an die Gefäßwand zu drücken. Außerdem verbliebe dann eine Verengung in diesem Bereich, die ebenfalls nicht gewünscht ist, da sie eine Strömungsquerschnittsreduzierung und damit eine Druckerhöhung bedeutet und somit gesundheitliche Nachteile für den Patienten mit sich bringt. Im Falle einer Herzklappeninsuffizienz kann die erfindungsgemäße implantierbare Einrichtung alternativ auch in der insuffizienten Klappe, beispielsweise Aortenklappe oder Mitralklappe, implantiert werden, ohne dass die natürliche Klappe vorher entfernt wird.

Beispielsweise kann die erfindungsgemäße implantierbare Einrichtung über die Aorta carotis oder Arteria axillaris in den Körper eingeführt werden, was im Vergleich zu einer Implantation über die Leistengegend des Patienten zu einem verkürzten Implantationsweg führt.

Der Grundkörper der implantierbaren Einrichtung wird vorzugsweise so ausgerichtet, dass der am ersten Ende auskragende Verankerungsabschnitt in die Herzkammer, beispielsweise die linke Herzkammer, hineinragt und der übrige Bereich des Grundkörpers sich an einer Gefäßwand, beispielsweise der Aortenwand, festklemmt. Hierdurch wird ein besonders guter Halt und eine stabile Anordnung ermöglicht. Die Abmessungen des wenigstens einen Verankerungsabschnitts und des Grundkörpers können dabei individuell an den Patienten angepasst werden, in Abhängigkeit von der Anatomie des jeweiligen Patienten. Auch das Maß des Auskragens des wenigstens einen Verankerungsabschnitts kann individuell gewählt werden. Grundsätzlich ist jedoch auch eine Standardisierung möglich, bei der die Verankerungsabschnitte in einem solchen Maß auskragen und solche Abmessungen aufweisen, dass der größte Teil der Patienten mit dieser Art von Grundkörper bzw. implantierbaren Einrichtung versorgt werden kann.

Nach einer Variante der Erfindung sind die mehreren miteinander verbundenen drahtartigen Elemente aus einem einzelnen Draht, einer Drahtlitze aus wenigstens zwei einzelnen Drähten und/oder einem Mehrfachdraht gebildet, insbesondere ist die implantierbare Einrichtung in Bereichen mit unterschiedlicher Steifigkeit aus unterschiedlichen Elementen, wie insbesondere einem einzelnen Draht, einer Drahtlitze aus wenigstens zwei einzelnen Drähten und/oder einem Mehrfachdraht ausgebildet.

Das einzige drahtartige Element der erfindungsgemäßen implantierbaren Einrichtung ist in einer erfindungsgemäßen Variante aus einem einzelnen Draht, einer Drahtlitze aus wenigstens zwei einzelnen Drähten oder einem Mehrfachdraht gebildet. Der Drahtquerschnitt kann beispielsweise rund, oval, halbrund, quadratisch oder rechteckig sein und auch über die Länge des drahtartigen Elements variieren, insbesondere in Bereichen mit unterschiedlicher Steifigkeit. Das einzige drahtartige Element kann gegebenenfalls mit Platin oder Gold oder Wolfram umwickelt oder mit Platin- oder Goldringen versehen sein, um den Röntgenkontrast zu erhöhen. Im Falle einer Drahtlitze können vorzugsweise Einzeldrähte aus Platin oder Gold zur Erhöhung der Röntgensichtbarkeit in die Litze eingearbeitet werden. Insbesondere die Verwendung einer Drahtlitze oder eines Mehrfachdrahts hat den Vorteil, dass die implantierbare Vorrichtung gemäß der Erfindung besonders flexibel bei ausreichender Stabilität ist.

Gemäß der Erfindung wird der Grundkörper durch verschränkendes Wickeln und/oder Verwinden und/oder Verflechten nach Art eines Gewebes und/oder Geleges und/oder Netzes geformt. Durch das verschränkende Wickeln und/oder Verwinden und/oder Verflechten des einzigen drahtartigen Elements entsteht in dem ersten Betriebszustand (Primärform) im Wesentlichen ein schlauchförmiges Element. Die beiden Enden des einzigen drahtartigen Elements sind an dem ersten Ende oder dem zweiten Ende oder in einer Seitenfläche des Grundkörpers angeordnet. Hierdurch verringert sich zum Einen das Verletzungsrisiko für den Patienten, bei dem die implantierbare Einrichtung implantiert wird und zum Anderen verbessert sich die Stabilität des Grundkörpers.

Nach einer Variante der erfindungsgemäßen implantierbaren Einrichtung ist der wenigstens eine Verankerungsabschnitt in dem zweiten Betriebszustand (Sekundärform) scheibenförmig oder schirmförmig ausgebildet. Alternativ ist der eine oder ein weiterer Verankerungsabschnitt bauchig und/oder gekrümmt ausgebildet. Nach der Implantation der erfindungsgemäßen Einrichtung liegt der scheibenförmige oder schirmförmige ausgebildete Verankerungsabschnitt beispielsweise an der Innenwandung des Herzens an und der verbleibende zylinderförmige Teil des Grundkörpers erstreckt sich durch die Öffnung in ein Gefäß, welche durch die implantierbare Einrichtung mit einer Klappeneinrichtung versehen werden soll. Damit sich der scheibenförmige oder schirmförmige Verankerungsabschnitt besser an die Innenwandung des Herzens anlegt, ist dieser teilweise deformierbar ausgebildet.

In einer weiteren Variante der erfindungsgemäßen implantierbaren Einrichtung weist der Grundkörper in dem zweiten Betriebszustand (Sekundärform) zwei sich jeweils in radialer Richtung des Grundkörpers erstreckende Verankerungsabschnitte auf. Beispielsweise ist der erste Verankerungsabschnitt an dem ersten Ende und der zweite Verankerungsabschnitt an dem zweiten Ende des Grundkörpers in dessen zweiten Betriebszustand (Sekundärform) angeordnet. Dabei weist der Grundkörper zwischen dem ersten und dem zweiten Verankerungsabschnitt einen Zwischenabschnitt auf, welcher einen geringeren Durchmesser aufweist als der erste Verankerungsabschnitt und der zweite Verankerungsabschnitt. Der Durchmesser der zwei Verankerungsabschnitte ist gleich groß oder unterschiedlich. Eine implantierbare Einrichtung mit einem ersten Verankerungsabschnitt und einem zweiten Verankerungsabschnitt, wobei die Verankerungsabschnitte unterschiedlich groß sind, kann beispielsweise derart ausgebildet sein, dass sich der erste Verankerungsabschnitt in eine Herzkammer erstreckt und dort in dem zweiten Betriebszustand (Sekundärform) an der Innenwandung der Herzens festhält und der zweite Verankerungsabschnitt in dem zweiten Betriebszustand (Sekundärform) an der Innenwandung eines Gefäßes, zum Beispiel der Aorta, festhält. Der erste Verankerungsabschnitt ist somit größer ausgebildet als der zweite Verankerungsabschnitt, da innerhalb der Herzkammer mehr Raum zur Verfügung steht als in dem Gefäß. Eine implantierbare Einrichtung mit zwei Verankerungsabschnitten, wobei die beiden Verankerungsabschnitte gleich groß ausgebildet sind, kann beispielsweise in einer Wandung eines Organs oder eines Gefäßes eingesetzt werden, wobei die Öffnung durch die Klappeneinrichtung in eine Flussrichtung verschlossen wird und in der anderen Flussrichtung eine Fluidverbindung zur Verfügung stellt. Eine derartige Ausgestaltung, beispielsweise als Doppelschirm, ermöglicht einen festen Sitz in der Öffnung des Organs oder des Gefäßes.

Gemäß der Erfindung weist der wenigstens eine Verankerungsabschnitt in dem zweiten Betriebszustand (Sekundärform) des Grundkörpers einen ersten Teilabschnitt und einen zweiten Teilabschnitt auf, wobei sich der erste Teilabschnitt in radialer Richtung des Grundkörpers nach außen erstreckt und der zweite Teilabschnitt in radialer Richtung des Grundkörpers nach innen zurückgefaltet ist, insbesondere derart, dass der erste Teilabschnitt und der zweite Teilabschnitt doppellagig aufeinander gefaltet sind. Erfindungsgemäß ist die Zurückfaltung des wenigstens einen Verankerungsabschnitts zur Mitte des Grundkörpers hin angeordnet. Der Verankerungsabschnitt weist somit eine Zurückfaltung auf, die in Richtung der Mitte des Grundkörpers angeordnet ist. Dadurch wird ein noch besserer Halt der erfindungsgemäßen Einrichtung erzielt. Weiterhin ist der Verankerungsabschnitt durch die Zurückfaltung und die dadurch erzielte Doppellagigkeit, in diesem Bereich zumindest teilweise deformierbar ausgebildet, so dass sich der Verankerungsabschnitt besser an eine Organ- oder Gefäßwandung anlegen kann.

In einer zweckmäßigen Variante der implantierbaren Einrichtung ist die Materialkonzentration und/oder die Materialstärke innerhalb der implantierbaren Einrichtung abschnittsweise unterschiedlich. In einer bevorzugten Variante ist die Materialmenge im Randbereich der implantierbaren Einrichtung und/oder im Randbereich des wenigstens einen Verankerungsabschnitts an die gewünschten mechanischen Eigenschaften angepasst, insbesondere ist eine Materialkonzentration im Randbereich der implantierbaren Einrichtung und/oder im Randbereich des wenigstens einen Verankerungsabschnitts zur partiellen Versteifung vorgesehen.

Gemäß einer Variante der implantierbaren Einrichtung weist das erste Ende und/oder das zweite Ende des Grundköpers eine oder mehrere miteinander verschränkte und/oder nebeneinander angeordnete und/oder ineinander verschlungene Schlingen oder Schlaufen auf. Diese Schlingen oder Schlaufen können einen gleichmäßigen oder ungleichmäßigen Rand ausbilden. Ein gleichmäßiger Rand wird beispielsweise durch Schlingen oder Schlaufen gleicher Größe gebildet und ein ungleichmäßiger Rand durch Schlingen oder Schlaufen unterschiedlicher Größe, zum Beispiel mit zwei unterschiedlichen Größen. Zweckmäßigerweise ist die Anordnung der unterschiedlich großen Schlingen oder Schlaufen gleichmäßig, beispielsweise eine große Schlinge oder Schlaufe nach drei kleinen Schlingen oder Schlaufen. Die implantierbare Einrichtung kann somit an die Implantationsstelle des Patienten angepasst werden. Ein ungleichmäßiger Rand hat zum Beispiel den Vorteil, dass sich die größeren Schlingen oder Schlaufen in den Kapillarmuskel des Herzens erstrecken und dort festhalten, wodurch ein besserer Halt der erfindungsgemäßen implantierbaren Einrichtung erzielt wird.

Zweckmäßigerweise ist der Grundkörper der implantierbaren Einrichtung nach einer Variante in dem ersten Betriebszustand (Primärform) Stent-förmig ausgebildet. Dadurch lässt sich die erfindungsgemäße implantierbare Einrichtung auf einfache Art und Weise durch einen Katheter implantieren.

In einer zweckmäßigen Variante der erfindungsgemäßen implantierbaren Einrichtung sind die beiden Enden des drahtartigen Elements miteinander geeignet verbindbar oder verbunden, insbesondere durch Auffügen eines weiteren Elements, durch Verdrehen, Verkleben, Schweißen, Löten oder einem anderen Verbindungsverfahren. Dadurch, dass die beiden Enden des drahtartigen Elements miteinander verbindbar oder verbunden sind, kann sichergestellt werden, dass keines der beiden Enden nach der Implantation der implantierbaren Einrichtung gemäß der Erfindung ein umliegendes Gefäß oder Organ verletzen können.

Nach einer zweckmäßigen Variante der erfindungsgemäßen implantierbaren Einrichtung weist die implantierbare Einrichtung in dem ersten Betriebszustand (Primärform) einen runden und/oder ovalen Querschnitt auf. In dem zweiten Betriebszustand (Sekundärform) der implantierbaren Einrichtung weist diese wenigstens in den Bereichen, wo kein Verankerungsabschnitt angeordnet ist, vorzugsweise ebenfalls in den Bereichen des wenigstens einen Verankerungsabschnitts, einen runden oder ovalen Querschnitt auf.

Zweckmäßigerweise ist der Durchmesser der implantierbaren Einrichtung in dem zweiten Betriebszustand (Sekundärform) in Bereichen außerhalb des Verankerungsabschnitts ungefähr 35 mm. Die Länge der erfindungsgemäßen implantierbaren Einrichtung in dem zweiten Betriebszustand (Sekundärform) ist vorzugsweise maximal 50 mm.

Nach einer Variante der erfindungsgemäßen implantierbaren Einrichtung weist der Grundkörper eine oder mehrere Lagen auf. Dadurch kann beispielsweise die Stabilität des Grundkörpers oder die Fluiddichtigkeit des Grundkörpers angepasst werden.

Nach einer weiteren Variante der erfindungsgemäßen implantierbaren Einrichtung sind der Grundkörper und das erste Membranelement einstückig ausgebildet. Dies kann beispielsweise dadurch erreicht werden, dass sich das erste Ende des Grundkörpers oder das zweite Ende des Grundkörpers in dem zweiten Betriebszustand (Sekundärform) nach innen erstreckt, wobei das erste Ende des Grundkörpers bzw. das zweite Ende des Grundkörpers ein Klappenelement in der ersten Öffnung des Grundkörpers bzw. der zweiten Öffnung des Grundkörpers ausbilden.

Nachfolgend wird die Erfindung anhand von in den Figuren dargestellten Ausführungsbeispielen näher erläutert. Es zeigen:
- Figur 1: eine perspektivische Ansicht einer ersten Ausführungsform einer nicht erfindungsgemäßen implantierbaren Einrichtung,
- Figur 2: eine Schnittansicht der ersten Ausführungsform der nicht erfindungsgemäßen implantierbaren Einrichtung aus Figur 1,
- Figur 3: alternative Ausführungen der implantierbaren Einrichtung aus Figur 1,
- Figur 4: eine perspektivische Ansicht einer zweiten Ausführungsform einer erfindungsgemäßen implantierbaren Einrichtung,
- Figur 5: eine Draufsicht auf das erste Ende der implantierbaren Einrichtung aus Figur 4,
- Figur 6: eine perspektivische Ansicht einer dritten Ausführungsform einer erfindungsgemäßen implantierbaren Einrichtung,
- Figur 7: eine Draufsicht auf das erste Ende der implantierbaren Einrichtung aus Figur 6,
- Figur 8: eine perspektivische Ansicht einer vierten Ausführungsform einer erfindungsgemäßen implantierbaren Einrichtung,
- Figur 9: eine Draufsicht auf das erste Ende der implantierbaren Einrichtung aus Figur 8,
- Figur 10: eine perspektivische Ansicht einer fünften Ausführungsform einer erfindungsgemäßen implantierbaren Einrichtung,
- Figur 11: eine perspektivische Ansicht einer sechsten Ausführungsform einer erfindungsgemäßen implantierbaren Einrichtung,
- Figur 12: eine perspektivische Ansicht einer siebten Ausführungsform einer erfindungsgemäßen implantierbaren Einrichtung,
- Figur 13: eine perspektivische Ansicht einer achten Ausführungsform einer erfindungsgemäßen implantierbaren Einrichtung,
- Figur 14: eine Detailansicht eines einzelnen Drahts zur Ausbildung eines Drahtelements einer erfindungsgemäßen implantierbaren Einrichtung,
- Figur 15: eine Detailansicht eines Mehrfachdrahts zur Ausbildung eines Drahtelements einer erfindungsgemäßen implantierbaren Einrichtung,
- Figur 16: Detailansichten von Drahtlitzen zur Ausbildung eines Drahtelements einer erfindungsgemäßen implantierbaren Einrichtung,
- Figur 17: eine Schnittansicht einer neunten Ausführungsform einer erfindungsgemäßen implantierbaren Einrichtung,
- Figur 18: eine Schnittansicht einer zehnten Ausführungsform einer erfindungsgemäßen implantierbaren Einrichtung,
- Figur 19: eine Schnittansicht einer elften Ausführungsform einer erfindungsgemäßen implantierbaren Einrichtung,
- Figur 20: eine Schnittansicht einer zwölften Ausführungsform einer erfindungsgemäßen implantierbaren Einrichtung im implantierten Zustand,
- Figur 21: eine Schnittansicht einer dreizehnten Ausführungsform einer nicht erfindungsgemäßen implantierbaren Einrichtung,
- Figur 22: eine Schnittansicht einer vierzehnten Ausführungsform einer nicht erfindungsgemäßen implantierbaren Einrichtung,
- Figur 23: eine Schnittansicht einer fünfzehnten Ausführungsform einer nicht erfindungsgemäßen implantierbaren Einrichtung,
- Figur 24: eine Schnittansicht einer sechszehnten Ausführungsform einer nicht erfindungsgemäßen implantierbaren Einrichtung,
- Figur 25: eine Schnittansicht einer siebzehnten Ausführungsform einer nicht erfindungsgemäßen implantierbaren Einrichtung,
- Figur 26: eine Schnittansicht einer achtzehnten Ausführungsform einer nicht erfindungsgemäßen implantierbaren Einrichtung,
- Figur 27: eine Schnittansicht einer neunzehnten Ausführungsform einer nicht erfindungsgemäßen implantierbaren Einrichtung,
- Figur 28: eine Schnittansicht einer zwanzigsten Ausführungsform einer nicht erfindungsgemäßen implantierbaren Einrichtung,
- Figur 29: eine Schnittansicht einer einundzwanzigsten Ausführungsform einer nicht erfindungsgemäßen implantierbaren Einrichtung,
- Figur 30: eine Schnittansicht einer zweiundzwanzigsten Ausführungsform einer nicht erfindungsgemäßen implantierbaren Einrichtung,
- Figur 31: eine Detailansicht eines Grundkörpers einer implantierbaren Einrichtung mit zwei Lagen,
- Figur 32: eine Detailansicht eines Grundkörpers einer implantierbaren Einrichtung mit drei Lagen,
- Figur 33: einen beispielshaften implantationsvorgang einer erfindungsgemäßen implantierbaren Einrichtung,
- Figur 34: eine Detailansicht einer ersten Ausgestaltung eines Randbereichs einer implantierbaren Einrichtung,
- Figur 35: eine Detailansicht einer zweiten Ausgestaltung eines Randbereichs einer implantierbaren Einrichtung, und
- Figur 36: eine Detailansicht einer dritten Ausgestaltung eines Randbereichs einer implantierbaren Einrichtung.

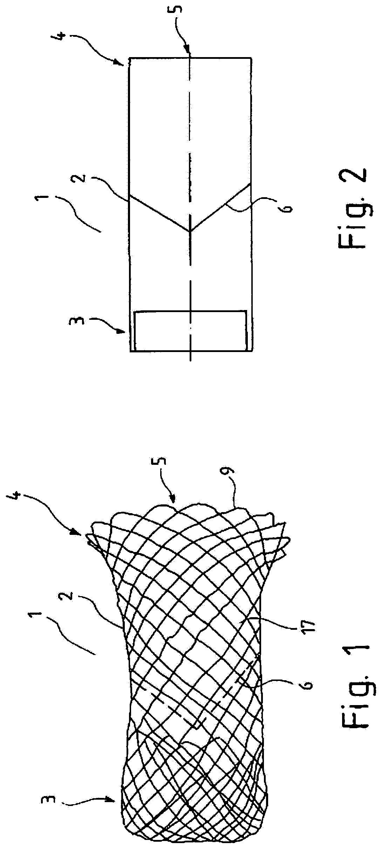

In Figur 1 ist eine perspektivische Ansicht einer ersten Ausführungsform einer nicht erfindungsgemäßen implantierbaren Einrichtung 1 zur Verwendung im menschlichen oder tierischen Körper 24 zum Ersatz einer Organklappe dargestellt. Die implantierbare Einrichtung 1 umfasst einen Grundkörper 2 mit einem ersten Ende 3 und einem zweiten Ende 4, wobei das erste Ende 3 und das zweite Ende 4 jeweils eine Öffnung 5 aufweisen, um eine Fluidverbindung zwischen dem ersten Ende 3 und dem zweiten Ende 4 durch den Grundkörper 2 bereitzustellen. Der Grundkörper 2 hat in einem ersten Betriebszustand (Primärform) ein großes Verhältnis von Länge zu Querausdehnung entlang der Längsachse des Grundkörpers 2 und in einem zweiten Betriebszustand (Sekundärform) ein kleineres Verhältnis von Länge zu Querausdehnung entlang der Längsachse des Grundkörpers 2, wobei die implantierbare Einrichtung 1 in Figur 1 in dem zweiten Betriebszustand (Sekundärform) dargestellt ist.

Der Grundkörper 2 der implantierbaren Einrichtung 1 ist durch Aufbringen einer Kraft gegen elastische Materialkräfte aus der Sekundärform in die Primärform reversibel überführbar.

Mittig zwischen dem ersten Ende 3 des Grundkörpers 2 und des zweiten Endes 4 des Grundkörpers 2 der implantierbaren Einrichtung 1 aus Figur 1 ist ein erstes Membranelement 6 angeordnet, wobei das Membranelement 6 derart ausgebildet, dass es die Fluidverbindung durch den Grundkörper 2 in eine erste Flussrichtung freigibt und in eine zweite Flussrichtung, entgegengesetzt zu der ersten Flussrichtung sperrt.

Der Grundkörper 2 der implantierbaren Einrichtung 1 aus Figur 1 ist aus einem einzigen drahtartigem Element 9 oder mehreren miteinander verbundenen drahtartigen Elementen 9 durch verschränkendes Wickeln und/oder Verwinden und/oder Verflechten nach Art eines Gewebes und/oder Geleges und/oder Netzes geformt.

Die implantierbare Einrichtung 1 aus Figur 1 ist in dem zweiten Betriebszustand (Sekundärform) stentförmig ausgebildet. Das erste Ende 3 des Grundkörpers 2 der implantierbaren Einrichtung 1 aus Figur 1 ist in dem zweiten Betriebszustand (Sekundärform) doppellagig aufeinander gefaltet, wobei die Doppellagigkeit des ersten Endes 3 durch eine Zurückfaltung nach innen in die implantierbare Einrichtung 1 ausgebildet ist.

Dadurch wird eine implantierbare Einrichtung 1 zum Ersatz einer Orangklappe geschaffen, mittels derer aufgrund der Doppellagigkeit an dem ersten Ende 3 des Grundkörpers 2 ein besonders guter Halt in ein Gefäß und/oder Organ möglich ist. Da der Grundkörper 2 reversibel von der Primärform in die Sekundärform überführbar ist, kann er problemlos durch einen Katheter 26 an den Implantationsort gebracht werden. Beim Herausschieben aus dem Katheter 26 entfaltet sich die implantierbare Einrichtung 1 aus der Primärform in die Sekundärform, wobei sich der Durchmesser des Grundkörpers 2 vergrößert und dadurch im Allgemeinen die Länge verringert. Durch die Möglichkeit des reversiblen Überführens der Primär- in die Sekundärform und umgekehrt, der Sekundär- in die Primärform, kann auch ein Zurückziehen der implantierbaren Einrichtung 1 in den Katheter 26 erfolgen, sofern während der Implantation festgestellt wird, dass diese nicht ordnungsgemäß verläuft, also insbesondere die implantierbare Einrichtung 1 nicht ordnungsgemäß zu den Abgängen der Koronargefäße und/oder der natürlichen Herzklappe und/oder der Aorta sowie der Herzkammer positioniert ist. Bei der Verwendung eines einzigen drahtartigen Elements 9, welches durch verschränkendes Wickeln und/oder Verwinden und/oder Verflechten nach Art eines Gewebes und/oder Geleges und/oder Netzes die implantierbare Einrichtung 1 ausformt, ergibt sich der Vorteil, dass die erfindungsgemäße implantierbare Einrichtung 1 eine hohe Flexibilität aufweist, ohne dass die Gefahr besteht, dass einzelne Elemente der implantierbaren Einrichtung 1 bei der Verformung von der Primärform in die Sekundärform bzw. von der Sekundärform in die Primärform brechen. Die Ausbildung der implantierbaren Einrichtung 1, insbesondere des Grundkörpers 2, aus einem einzigen drahtartigen Element 9 hat weiterhin den Vorteil, dass keine Verbindungspunkte, beispielsweise Schweißpunkte, zwischen den einzelnen Elementen des Grundkörpers 2 vorhanden sind, die besonders leicht brechen könnten. Gerade ein solches Auseinanderbrechen von einzelnen Elementen einer implantierbaren Einrichtung 1 kann dazu führen, dass scharfkantige Bereiche aus einer Vorrichtung herausragen, die die Wand, insbesondere die Aorta, verletzen bzw. perforieren können. Die Struktur des Grundkörpers 2 kann außerdem bei einstückiger Ausbildung gleichmäßiger gestaltet werden, als dies beim Zusammenfügen von einzelnen ringförmigen Elementen, wie aus dem Stand der Technik bekannt, möglich ist. Die Verwendung von mehreren miteinander verbundenen drahtartigen Elementen 9 hat den Vorteil, dass die implantierbare Einrichtung 1 einfacher maschinell herstellbar ist.

Die implantierbare Einrichtung 1 aus Figur 1 umfasst mindestens eine röntgendichte Markierung, nicht dargestellt, insbesondere in Form von Markerplaketten, Markierungen oder Markerdrähten. Die röntgendichte Markierung ist insbesondere im Bereich des Grundkörpers 2 angeordnet. Hierdurch ist es möglich, während des Implantationsvorgangs die Positionierung, insbesondere hinsichtlich der Abgänge zu Koronargefäßen über einen Monitor oder dergleichen zu verfolgen. Hierzu eignen sich insbesondere die Röntgendarstellung oder Magnetresonanztomographie, die eine achsgenaue Position der implantierbaren Einrichtung 1, die insbesondere einen Aortenklappenersatz ist, im Körper des Patienten wiedergeben können. Die Markierungen können an verschiedenen Stellen des Grundkörpers 2 bzw. der implantierbaren Einrichtung 1, insbesondere in Bereichen von Öffnungen im Grundkörper 2 vorgesehen werden.

Die in Figur 1 dargestellte implantierbare Einrichtung 1 besteht aus einem Formgedächtnismaterial, insbesondere Nitinol oder einem Kunststoff mit Formgedächtnis. Je nach Anwendungsfall der implantierbaren Einrichtung 1 kann diese ganz oder teilweise aus einem resorbierbaren Material bestehen, insbesondere einem resorbierbaren Kunststoff mit Formgedächtnis.

Das erste Membranelement 6 der implantierbaren Einrichtung 1 aus Figur 1 besteht aus einem synthetischen oder biologischen Material, insbesondere aus Polyurethan. Das erste Membranelement 6 ist weiterhin mit einer Beschichtung versehen, zum Schaffen einer Biostabilität, insbesondere einer Titanbeschichtung.

Der Grundkörper 2 und das erste Membranelement 6 können lösbar oder unlösbar miteinander verbindbar oder verbunden sein, insbesondere durch Kleben, Schweißen, Nähen, Aufschmelzen, Tauchen oder einem anderen Fügeverfahren.

Das erste Membranelement 6 ist im zweiten Betriebszustand (Sekundärform) der implantierbaren Einrichtung 1 mittig zwischen dem ersten Ende 3 des Grundkörpers 2 und dem zweiten Ende 4 des Grundkörpers 2 angeordnet.

Dadurch, dass der Grundkörper 2 durch Aufbringen einer Kraft gegen elastische Materialkräfte aus der Sekundärform in die Primärform reversibel überführbar ist, ist die implantierbare Einrichtung 1 gemäß Figur 1 replazierbar und explantierbar.

Die implantierbare Einrichtung 1 ist im zweiten Betriebszustand (Sekundärform), wie in Figur 1 dargestellt, zwischen dem ersten Ende 3 und dem zweiten Ende 4 des Grundkörpers 2 mindestens teilweise, vorzugsweise vollständig in radialer Richtung deformierbar ausgebildet, so dass sich der Grundkörper 2 an eine Gefäßwandung anlegen kann. Dadurch, dass sich der Grundkörper 2 bei der Implantation an eine Gefäßwandung anlegt, wird erreicht, dass sich die implantierbare Einrichtung 1 nach der Implantation nicht selbstständig, beispielsweise verursacht durch die Pumpbewegung des Herzens, replaziert. Die implantierbare Einrichtung 1 aus Figur 1 ist beispielsweise über die Aorta carotis oder arteria axelliaris in den menschlichen oder tierischen Körper 24 einführbar, was im Vergleich zu einer Implantation über die Leistengegend des Patienten zu einem verkürzten Implantationsweg führt.

Bei der Verwendung eines einzigen drahtartigen Elementes 9 zur Ausbildung des Grundkörpers 2 der implantierbaren Einrichtung 1 wird dieses beispielsweise durch einen einzelnen Draht 17, einer Drahtlitze 18 aus wenigstens zwei einzelnen Drähten oder einem Mehrfachdraht 19 ausgebildet. Der Drahtquerschnitt kann beispielsweise rund, oval, halbrund, quadratisch oder rechteckig sein und auch über die Länge des einzigen drahtartigen Elements 9 variieren. Das drahtartige Element 9 kann gegebenenfalls mit Platin oder Gold oder Wolfram umwickelt oder mit Platin- oder Goldringen versehen sein, um den Röntgenkontrast zu erhöhen. Im Falle einer Drahtlitzte 18 können vorzugsweise Einzeldrähte aus Platin oder Gold zur Erhöhung der Röntensichtbarkeit in die Drahtlitze 18 eingearbeitet werden. Insbesondere die Verwendung einer Drahtlitze 18 oder eines Mehrfachdrahtes 19 hat den Vorteil, dass die implantierbare Einrichtung 1 besonders flexibel bei ausreichender Stabilität ist.

Der Grundkörper 2 wird durch verschränkendes Wickeln und/oder Verwinden und/oder Verflechten nach Art eines Gewebes und/oder Geleges und/oder Netzes geformt. Durch das verschränkende Wickeln und/oder Verwinden und/oder Verflechten des einzigen drahtartigen Elements 9 oder der mehreren miteinander verbundenen drahtartigen Elementen 9 entsteht in dem ersten Betriebszustand (Primärform) im Wesentlichen ein schlauchförmiges oder zylinderförmiges Element. Die beiden Enden des einzigen drahtartigen Elements 9 sind beispielsweise an dem ersten Ende 3 oder dem zweiten Ende 4 oder in einer Seitenfläche des länglichen Grundkörpers 2 angeordnet.

Wie der Figur 1 weiterhin entnommen werden kann, weist die implantierbare Einrichtung 1 in dem zweiten Betriebszustand (Sekundärform) einen runden Querschnitt auf.

In Figur 2 ist eine Schnittansicht der ersten Ausführungsform der implantierbaren Einrichtung 1 aus Figur 1 dargestellt. Figur 2 ist insbesondere zu entnehmen, dass an dem ersten Ende 3 des Grundkörpers 2 der implantierbaren Einrichtung 1 eine Doppellagigkeit durch einen nach innen in die implantierbare Einrichtung hinein erfolgte Zurückfaltung angeordnet ist. Durch die Doppellagigkeit an dem ersten Ende 3 wird die Verankerung der implantierbaren Einrichtung 1 in dem zweiten Betriebszustand (Sekundärform) an dem Implantationsort verbessert.

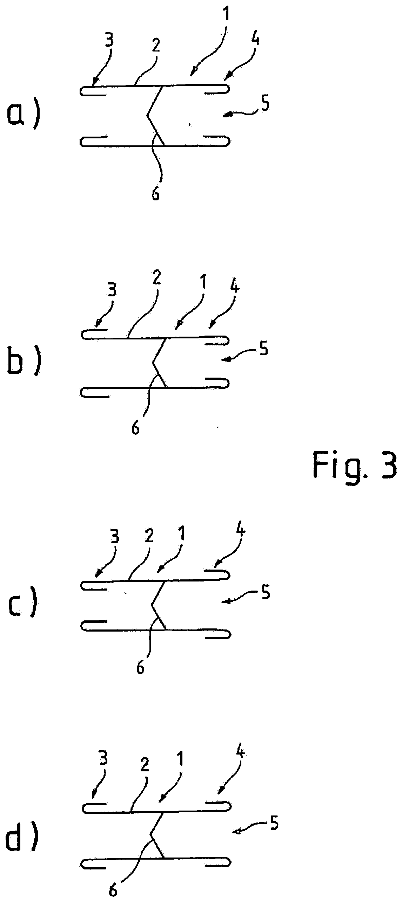

In Figur 3a sind alternative Ausführungen der implantierbaren Einrichtung 1 aus Figur 1 dargestellt. Die dargestellten alternativen Ausführungen unterscheiden sich hinsichtlich der Zurückfaltung an dem ersten Ende 3 und/oder dem zweiten Ende 4 des Grundkörpers 2 der implantierbaren Einrichtung 1.

In Figur 3b sind sowohl das erste Ende 3 wie auch das zweite Ende 4 des Grundkörpers 2 der implantierbaren Einrichtung 1 doppellagig durch eine Zurückfaltung nach innen in die implantierbare Einrichtung 1 hinein ausgebildet.

In Figur 3 ist das erste Ende 3 des Grundkörpers 2 der implantierbaren Einrichtung 1 doppellagig durch eine Zurückfaltung nach außen an den Grundkörper 2 ausgebildet und das zweite Ende 4 des Grundkörpers 2 der implantierbaren Einrichtung 1 ist doppellagig durch eine Zurückfaltung in die implantierbare Einrichtung 1 hinein ausgebildet.

Gemäß Figur 3c ist das erste Ende 3 des Grundkörpers 2 der implantierbaren Einrichtung 1 nach innen in die implantierbare Einrichtung 1 hinein zurückgefaltet, zur Ausbildung einer Doppellagigkeit und das zweite Ende 4 ist nach außen an den Grundkörper zurückgefaltet zur Ausbildung einer Doppellagigkeit.

Gemäß Figur 3d sind das erste Ende 3 und das zweite Ende 4 des Grundkörpers 2 der implantierbaren Einrichtung 1 nach außen an den Grundkörper 2 zurückgefaltet, zur Ausbildung einer Doppellagigkeit an dem ersten Ende 3 und dem zweiten Ende 4 des Grundkörpers 2 der implantierbaren Einrichtung 1.

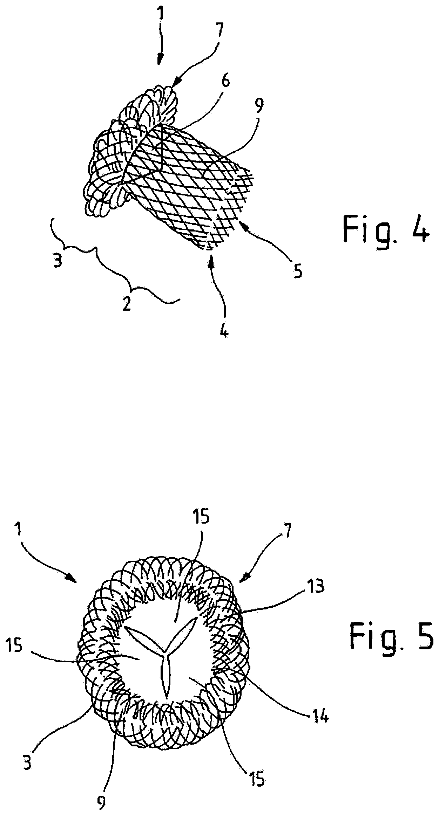

In Figur 4 ist eine perspektivische Ansicht einer zweiten Ausführungsform einer erfindungsgemäßen implantierbaren Einrichtung 1 zur Verwendung im menschlichen oder tierischen Körper 24 zum Ersatz einer Organklappe dargestellt. Die implantierbare Einrichtung 1 umfasst einen Grundkörper 2 mit einem ersten Ende 3 und einem zweiten Ende 4, wobei das erste Ende 3 und das zweite Ende 4 jeweils eine Öffnung 5 aufweisen, um eine Fluidverbindung zwischen dem ersten Ende 3 und dem zweiten Ende 4 durch den Grundkörper 2 bereitzustellen. Der Grundkörper 2 hat in einem ersten Betriebszustand (Primärform) ein großes Verhältnis von Länge zu Querausdehnung entlang der Längsachse des Grundkörpers 2 und in einem zweiten Betriebszustand (Sekundärform) ein kleineres Verhältnis von Länge zu Querausdehnung entlang der Längsachse des Grundkörpers 2. Die implantierbare Einrichtung 1 aus Figur 4 ist dabei in dem zweiten Betriebszustand (Sekundärform) dargestellt.

Der Grundkörper 2 der implantierbaren Einrichtung 1 ist durch Aufbringen einer Kraft gegen elastische Materialkräfte aus der Sekundärform in die Primärform reversibel überführbar.

Am ersten Ende 3 des Grundkörpers 2 der implantierbaren Einrichtung 1 aus Figur 4 ist ein erstes Membranelement 6 angeordnet, wobei das Membranelement 6 derart ausgebildet ist, dass es die Fluidverbindung durch den Grundkörper 2 in eine erste Flussrichtung freigibt und in eine zweite Flussrichtung, entgegengesetzt zu der ersten Flussrichtung, sperrt.

Weiterhin umfasst der Grundkörper 2 der implantierbaren Einrichtung 1 aus Figur 4 in dem zweiten Betriebszustand (Sekundärform) an dem ersten Ende 3 einen sich in radialer Richtung des Grundkörpers 2 erstreckenden Verankerungsabschnitt 7 zum Verankern der Einrichtung 1 in einem Organ und/oder einem Gefäß.

Der Grundkörper 2 der implantierbaren Einrichtung 1 aus Figur 4 ist aus einem einzigen drahtartigen Element 9 durch verschränkendes Wickeln und/oder Verwinden und/oder Verflechten nach Art eines Gewebes und/oder Geleges und/oder Netzes geformt.

Dadurch wird eine implantierbare Einrichtung 1 zum Ersatz einer Organklappe geschaffen, mittels derer aufgrund der Verwendung von dem auskragenden Verankerungsabschnitt 7 am ersten Ende 3 des Grundkörpers 2 ein besonders guter Halt in einem Gefäß und/oder Organ möglich ist. Bei der Verwendung der implantierbaren Einrichtung 1 gemäß Figur 4 als Herzklappenersatz kann das erste Ende 3 mit dem auskragenden Verankerungsabschnitt 7 beispielsweise in die linke Herzkammer hereinragen und sich dort festhalten und das zweite Ende 4 des Grundkörpers 2 an die Aortenwand anlegen. Da der Grundkörper 2 reversibel von der Primärform in die Sekundärform überführbar ist, kann er problemlos durch einen Katheter 26 an den Implantationsort gebracht werden. Beim Herausschieben aus dem Katheter 26 entfaltet sich die implantierbare Einrichtung 1 aus der Primärform in die Sekundärform, wobei sich der Durchmesser des Grundkörpers 2 vergrößert und dafür im Allgemeinen die Länge verringert. Durch die Möglichkeit des reversiblen Überführens der Primär- in die Sekundärform und umgekehrt, der Sekundär- in die Primärform, kann auch ein Zurückziehen der implantierbaren Einrichtung 1 in den Katheter 26 erfolgen, sofern während der Implantation festgestellt wird, dass diese nicht ordnungsgemäß verläuft, also insbesondere die implantierbare Einrichtung 1 nicht ordnungsgemäß zu den Abgängen der Koronargefäße und/oder der natürlichen Herzklappe und/oder der Aorta sowie der Herzkammer positioniert ist. Aufgrund der Verwendung eines einzigen drahtartigen Elements 9, welches durch verschränkendes Wickeln und/oder Verwinden und/oder Verflechten nach Art eines Gewebes und/oder Geleges und/oder Netzes die implantierbare Einrichtung 1 ausformt, ergibt sich der Vorteil, dass die erfindungsgemäße implantierbare Einrichtung 1 eine hohe Flexibilität aufweist, ohne dass die Gefahr besteht, dass einzelne Elemente der implantierbaren Einrichtung 1 bei der Verformung von der Primärform in die Sekundärform bzw. von der Sekundärform in die Primärform brechen. Die Ausbildung der implantierbaren Einrichtung 1, insbesondere des Grundkörpers 2, aus einem einzigen drahtartigen Element 9 hat weiterhin den Vorteil, dass keine Verbindungspunkte, beispielsweise Schweißpunkte, zwischen einzelnen Elementen des Grundkörpers 2 vorhanden sind, die besonders leicht brechen könnten. Gerade ein solches Auseinanderbrechen von einzelnen Elementen einer implantierbaren Einrichtung 1 kann dazu führen, dass scharfkantige Bereiche aus einer Vorrichtung herausragen, die die Wand, insbesondere die Aorta verletzen bzw. perforieren können. Die Struktur des Grundkörpers 2 kann außerdem bei einstückiger Ausbildung gleichmäßiger gestaltet werden, als dies beim Zusammenfügen von einzelnen ringförmigen Elementen, wie aus dem Stand der Technik bekannt, möglich ist.

Der sich in radialer Richtung des Grundkörpers 2 der implantierbaren Einrichtung 1 erstreckende Verankerungsabschnitt 7 ist um den Umfang des Grundkörpers 2 am ersten Ende 3 des Grundkörpers 2 umlaufend vorgesehen.

Die implantierbare Einrichtung 1 aus Figur 4 umfasst mindestens eine röntgendichte Markierung, nicht dargestellt, insbesondere in Form vom Markerplaketten, Markierungen oder Markerdrähten. Die röntgendichte Markierung ist insbesondere im Bereich des Grundkörpers 2 angeordnet. Hierdurch ist es möglich, während des Implantationsvorgangs die Positionierung, insbesondere hinsichtlich der Abgänge zu Koronargefäßen über einen Monitor oder dergleichen zu verfolgen. Hierzu eignen sich insbesondere die Röntgendarstellung oder Magnetresonanztomographie, die eine achsengenaue Position der implantierbaren Einrichtung 1, die insbesondere ein Aortenklappenersatz ist, im Körper des Patienten wiedergeben können. Die Markierungen können an verschiedenen Stellen des Grundkörpers 2 bzw. der implantierbaren Einrichtung 1, insbesondere in Bereichen von Öffnungen im Grundkörper 2, vorgesehen werden.

Die in Figur 4 dargestellte implantierbare Einrichtung 1 besteht aus einem Formgedächtnismaterial, insbesondere Nitinol oder einem Kunststoff mit Formgedächtnis. Je nach Anwendungsfall der implantierbaren Einrichtung 1 kann diese ganz oder teilweise aus einem resorbierbaren Material bestehen, insbesondere einem resorbierbaren Kunststoff mit Formgedächtnis.

Das erste Membranelement 6 der implantierbaren Einrichtung 1 aus Figur 4 besteht aus einem synthetischen oder biologischen Material, insbesondere aus Polyurethan. Das erste Membranelement 6 ist weiterhin mit einer Beschichtung versehen, zum Schaffen einer Biostabilität, insbesondere einer Titanbeschichtung.

Der Grundkörper 2 und das erste Membranelement 6 können lösbar oder unlösbar miteinander verbindbar oder verbunden sein, insbesondere durch Kleben, Schweißen, Nähen, Aufschmelzen, Tauchen oder einem anderen Fügeverfahren.

Das erste Membranelement 6 ist im zweiten Betriebszustand (Sekundärform) der implantierbaren Einrichtung 1 am ersten Ende 3 des Grundkörpers 2 angeordnet.

Dadurch, dass der Grundkörper 2 durch Aufbringen einer Kraft gegen elastische Materialkräfte aus der Sekundärform in die Primärform reversibel überführbar ist, ist die implantierbare Einrichtung 1 gemäß Figur 4 replazierbar und explantierbar.

Die implantierbare Einrichtung 1 ist im zweiten Betriebszustand (Sekundärform), wie in Figur 4 dargestellt, zwischen dem ersten Ende 3 und dem zweiten Ende 4 des Grundkörpers 2 wenigstens teilweise, vorzugsweise vollständig in radialer Richtung deformierbar ausgebildet, so dass sich der Grundkörper 2 an eine Gefäßwandung und/oder den Rand einer Öffnung und/oder den Rand einer defekten Organklappe anlegen kann. Dadurch, dass sich der Grundkörper 2 bei der Implantation an eine Gefäßwandung und/oder den Rand einer Öffnung und/oder den Rand einer defekten Organklappe anlegt, wird erreicht, dass sich die erfindungsgemäße implantierbare Einrichtung 1 nach der Implantation nicht selbstständig, beispielsweise verursacht durch die Pumpbewegung des Herzens, replaziert.

Beispielsweise wird die erfindungsgemäße implantierbare Einrichtung 1 während der Implantation in den menschlichen oder tierischen Körper 24 soweit in den Bereich einer Herzklappe, insbesondere einer Aortenklappe, eingeschoben, dass die natürliche Klappe während der Überführung der erfindungsgemäßen implantierbaren Einrichtung 1 von dem ersten Betriebszustand (Primärform) in den zweiten Betriebszustand (Sekundärform) an die Gefäßwand gedrückt und durch die implantierbare Einrichtung 1 dort gehalten wird. Hierbei wäre es grundsätzlich sogar möglich, in eine bereits implantierte Einrichtung 1 eine weitere implantierbare Einrichtung 1 einzufügen, wobei das erste Membranelement 6 der zuerst implantierten Einrichtung 1 dabei ebenfalls an die Wand des Grundkörpers 2 gedrückt würde. Ein solches Einfügen einer weiteren implantierbaren Einrichtung 1 in eine bereits implantierte Einrichtung 1 könnte sich beispielsweise bei nachlassender Stabilität und Beweglichkeit des ersten Membranelements 6 als sinnvoll erweisen. Auch ist es grundsätzlich möglich, nach vorherigem Entfernen einer alten natürlichen Klappe, insbesondere durch Operation, an dieser Stelle eine implantierbare Einrichtung 1 gemäß Figur 4 mit einem Membranelement 6 als Klappenersatz einzufügen. Gerade bei starker Verkalkung der natürlichen Herzklappe kann es sich als vorteilhaft erweisen, diese vollständig zu entfernen, da sie dann üblicherweise im Wesentlichen unbeweglich geworden ist. In diesem Fall wäre es ansonsten relativ schwer möglich, die natürliche Klappe an die Gefäßwand zu drücken. Außerdem verbliebe dann eine Verengung in diesem Bereich, die ebenfalls nicht gewünscht ist, da sie eine Strömungsquerschnittsreduzierung und damit eine Druckerhöhung bedeutet und somit gesundheitliche Nachteile für den Patienten mit sich bringt. Im Falle einer Herzklappeninsuffizienz kann die erfindungsgemäße implantierbare Einrichtung 1 alternativ auch in der insuffizienten Klappe, beispielsweise Aortenklappe oder Mitralklappe, implantiert werden, ohne dass die natürliche Klappe vorher entfernt wird.

Die erfindungsgemäße implantierbare Einrichtung 1 aus Figur 4 wird zum Beispiel über die Aorta karotis oder Arteria axillaris in den menschlichen oder tierischen Körper 24 eingeführt, was im Vergleich zu einer Implantation über die Leistengegend des Patienten zu einem verkürzten Implantationsweg führt.

Der Grundkörper 2 der implantierbaren Einrichtung 1 aus Figur 4 wird vorzugsweise so ausgerichtet, dass der am ersten Ende 3 auskragende Verankerungsabschnitt 7 in die Herzkammer beispielsweise die linke Herzkammer, hineinragt und der übrige Bereich des Grundkörpers 2 sich an einer Gefäßwand, beispielsweise der Aortenwand, festklemmt. Hierdurch wird ein besonders guter Halt und eine stabile Anordnung ermöglicht. Die Abmessungen des Verankerungsabschnitts 7 und des Grundkörpers 2 können dabei individuell an den Patienten angepasst werden, in Abhängigkeit von der Anatomie des jeweiligen Patienten. Auch das Maß des Auskragens des Verankerungsabschnitts 7 kann individuell gewählt werden. Grundsätzlich ist jedoch auch eine Standardisierung möglich, bei der der Verankerungsabschnitt 7 in einem solchen Maß auskragt und solche Abmessungen aufweist, dass der größte Teil der Patienten mit dieser Art von Grundkörper 2 bzw. implantierbarer Einrichtung 1 versorgt werden kann.

Das einzige drahtartige Element 9 der erfindungsgemäßen implantierbaren Einrichtung 1 ist aus einem einzelnen Draht 17, einer Drahtlitze 18 aus wenigstens zwei einzelnen Drähten oder einem Mehrfachdraht 19 gebildet. Der Drahtquerschnitt kann beispielsweise rund, oval, halbrund, quadratisch oder rechteckig sein und auch über die Länge des einzigen drahtartigen Elements 9 variieren. Das einzige drahtartige Element 9 kann gegebenenfalls mit Platin oder Gold oder Wolfram umwickelt oder mit Platin- oder Goldringen versehen sein, um den Röntgenkontrast zu erhöhen. Im Falle einer Drahtlitze 18 können vorzugsweise Einzeldrähte aus Platin oder Gold zur Erhöhung der Röntgensichtbarkeit in die Drahtlitze 18 eingearbeitet werden. Insbesondere die Verwendung einer Drahtlitze 18 oder eines. Mehrfachdrahts 19 hat den Vorteil, dass die implantierbare Einrichtung 1 gemäß der Erfindung besonders flexibel bei ausreichender Stabilität ist.

Gemäß der Erfindung wird der Grundkörper 2 durch verschränkendes Wickeln und/oder Verwinden und/oder Verflechten nach Art eines Gewebes und/oder Geleges und/oder Netzes geformt. Durch das verschränkende Wickeln und/oder Verwinden und/oder Verflechten des einzigen drahtartigen Elements 9 entsteht in dem ersten Betriebszustand (Primärform) im Wesentlichen ein schlauchförmiges oder zylinderförmiges Element. Die beiden Enden des einzigen drahtartigen Elements 9 sind an dem ersten Ende 3 oder dem zweiten Ende 4 oder in einer Seitenfläche des länglichen Grundkörpers 2 angeordnet. Hierdurch verringert sich zum Einen das Verletzungsrisiko für den Patienten, bei dem die implantierbare Einrichtung 1 implantiert wird und zum Anderen verbessert sich die Stabilität des Grundkörpers 2.

Der Verankerungsabschnitt 7 der implantierbaren Einrichtung 1 ist in dem zweiten Betriebszustand (Sekundärform) scheibenförmig ausgebildet, wie in Figur 4 dargestellt. Nach der Implantation der erfindungsgemäßen implantierbaren Einrichtung 1 liegt der scheibenförmig ausgebildete Verankerungsabschnitt 7 beispielsweise an der Innenwandung des Herzens an und der verbleibende zylinderförmige Teil des Grundkörpers 2 erstreckt sich durch die Öffnung, welche durch die implantierbare Einrichtung 1 mit einer Klappeneinrichtung versehen werden soll, in ein Gefäß. Damit sich der scheibenförmige Verankerungsabschnitt 7 besser an die Innenwandung des Herzens anlegt, ist dieser teilweise deformierbar ausgebildet.

Der Verankerungsabschnitt 7 weist in dem zweiten Betriebszustand (Sekundärform) des Grundkörpers 2 einen ersten Teilabschnitt 22 und einen zweiten Teilabschnitt 23 auf, wobei sich der erste Teilabschnitt 22 in radialer Richtung des Grundkörpers 2 nach außen erstreckt und der zweite Teilabschnitt 23 in radialer Richtung des Grundkörpers 2 nach innen zurückgefaltet ist, insbesondere derart, dass der erste Teilabschnitt 22 und der zweite Teilabschnitt 23 doppellagig aufeinander gefaltet sind. Die Zurückfaltung des Verankerungsabschnitts 7 ist zur Mitte des Grundkörpers 2 hin angeordnet. Der Verankerungsabschnitt 7 ist durch die Zurückfaltung und die dadurch erzielte Doppellagigkeit in diesem Bereich zumindest teilweise deformierbar ausgebildet, so dass sich der Verankerungsabschnitt 7 besser an eine Organ- oder Gefäßwandung anlegen kann.

Das erste Ende 3 und das zweite Ende 4 des Grundkörpers 2 weisen eine oder mehrere miteinander verschränkte und/oder nebeneinander angeordnete und/oder ineinander verschlungene Schlingen oder Schlaufen 21 auf. Diese Schlingen oder Schlaufen 21 bilden einen gleichmäßigen Rand 28 aus. Der gleichmäßige Rand 28 wird beispielsweise durch Schlingen oder Schlaufen 21 gleicher Größe gebildet.

Die beiden Enden des drahtartigen Elements 9 sind miteinander geeignet verbindbar oder verbunden, insbesondere durch Auffügen eines weiteren Elements, durch Verdrehen, Verkleben, Schweißen, Löten oder einem anderen Verbindungsverfahren. Dadurch wird sichergestellt, dass keines der beiden Enden nach der Implantation der implantierbaren Einrichtung 1 ein umliegendes Gefäß oder Organ verletzen können.

Wie der Figur 4 weiterhin entnommen werden kann, weist die implantierbare Einrichtung 1 in dem zweiten Betriebszustand (Sekundärform) wenigstens in den Bereichen, wo kein Verankerungsabschnitt 7 angeordnet ist, einen runden Querschnitt auf. Die implantierbare Einrichtung 1 aus Figur 4 ist ebenfalls im Bereich des Verankerungsabschnitts 7 mit einem runden Querschnitt versehen.

In Figur 5 ist eine Draufsicht auf das erste Ende 3 der implantierbaren Einrichtung 1 aus Figur 4 dargestellt. Der Figur 5 lässt sich insbesondere entnehmen, dass das erste Membranelement 6 einen Ringabschnitt 13 und einen mit diesem verbundenen Klappenabschnitt 14 aufweist. Der Klappenabschnitt 14 ist dabei durch drei Segelelemente 15 ausgebildet.

Das erste Membranelement 6 ist im zweiten Betriebszustand (Sekundärform) am ersten Ende 3 des Grundkörpers 2 angeordnet. Das erste Membranelement 6 ist beispielsweise im Bereich des Ringabschnitts 13 mit dem ersten Ende 3 des Grundkörpers 2 verbunden, beispielsweise durch Nähen. Der Klappenabschnitt 14 ist so dimensioniert, dass er die Fluidverbindung durch den Grundkörper 2 in eine erste Flussrichtung freigibt und in eine zweite Flussrichtung, entgegengesetzt zu der ersten Flussrichtung, sperrt, vorzugsweise derart, dass der Querschnitt des Klappenabschnitts 14 im Querschnitt dem Querschnitt der zu ersetzenden Klappenanordnung entspricht.

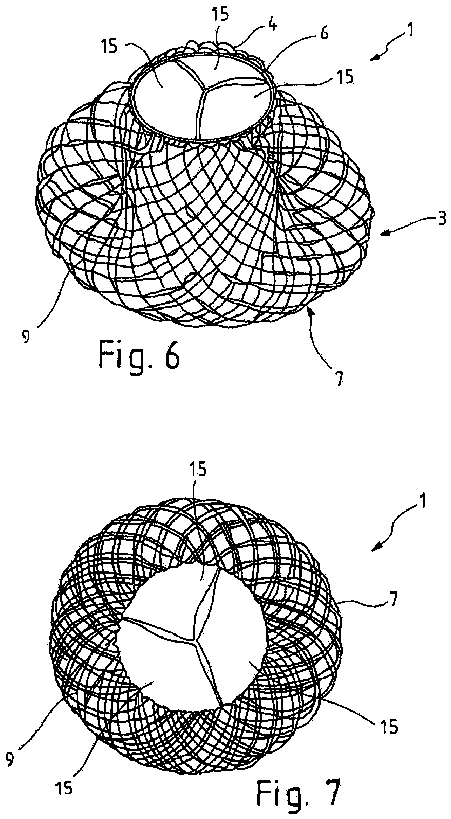

Figur 6 zeigt eine perspektivische Ansicht einer dritten Ausführungsform einer erfindungsgemäßen implantierbaren Einrichtung 1. Die implantierbare Einrichtung 1 zur Verwendung im menschlichen oder tierischen Körper 24 zum Ersatz einer Organklappe gemäß Figur 6 umfasst einen Grundkörper 2 mit einem ersten Ende 3 und einem zweiten Ende 4, wobei das erste Ende 3 und das zweite Ende 4 jeweils eine Öffnung 5 aufweisen, um eine Fluidverbindung zwischen dem ersten Ende 3 und dem zweiten Ende 4 durch den Grundkörper 2 bereitzustellen und ein erstes Membranelement 6 angeordnet am zweiten Ende 4 des Grundkörpers 2, wobei das Membranelement 6 derart ausgebildet ist, dass es die Fluidverbindung durch den Grundkörper 2 in eine erste Flussrichtung freigibt und in eine zweite Flussrichtung, entgegengesetzt zu der ersten Flussrichtung, sperrt. Der Grundkörper 2 weist in einem ersten Betriebszustand (Primärform) ein großes Verhältnis von Länge zu Querausdehnung entlang der Längsachse des Grundkörpers 2 und in einem zweiten Betriebszustand (Sekundärform) ein kleineres Verhältnis von Länge zu Querausdehnung entlang der Längsachse des Grundkörpers 2 auf. Der Grundkörper 2 ist durch Aufbringen einer Kraft gegen elastische Materialkräfte aus der Sekundärform in die Primärform reversibel überführbar. Die implantierbare Einrichtung 1 ist in Figur 6 in dem zweiten Betriebszustand (Sekundärform) dargestellt.

Der Grundkörper 2 weist in dem zweiten Betriebszustand (Sekundärform) an dem ersten Ende 3 einen sich in radialer Richtung des Grundkörpers 2 erstreckenden Verankerungsabschnitt 7 zum Verankern der Einrichtung 1 in einem Organ und/oder einem Gefäß auf.

Der Grundkörper 2 der implantierbaren Einrichtung 1 aus Figur 6 ist aus einem einzigen drahtartigem Element 9 oder mehreren miteinander verbundenen drahtartigen Elementen 9 durch verschränkendes Wickeln und/oder Verwinden und/oder Verflechten nach Art eines Gewebes und/oder Geleges und/oder Netzes geformt.

Die Ausführung gemäß Figur 6 unterscheidet sich von der Ausführung gemäß Figur 4 dadurch, dass das erste Membranelement 6 am zweiten Ende 4 des Grundkörpers 2 angeordnet ist. Weiterhin unterscheidet sich das erste Membranelement 6 gemäß der Ausführungsform aus Figur 6 von der Ausführungsform aus Figur 4 dadurch, dass dieses aus drei Segelelementen 15 gebildet wird und keinen Ringabschnitt 13 aufweist, wie das erste Membranelement 6 aus Figur 5.

In Figur 7 ist eine Draufsicht auf das erste Ende 3 der implantierbaren Einrichtung 1 aus Figur 6 dargestellt. Das erste Membranelement 6 der Ausführungsform aus Figur 7 ist somit an dem in der Zeichenebene hineinragenden Ende der implantierbaren Einrichtung 1 angeordnet und durch die in Längsrichtung durch den Grundkörper 2 verlaufende Fluidverbindung sichtbar. Im Übrigen entspricht die Ausgestaltung der Ausführungsform gemäß den Figuren 6 und 7 der Ausführungsform gemäß der Figuren 4 und 5.

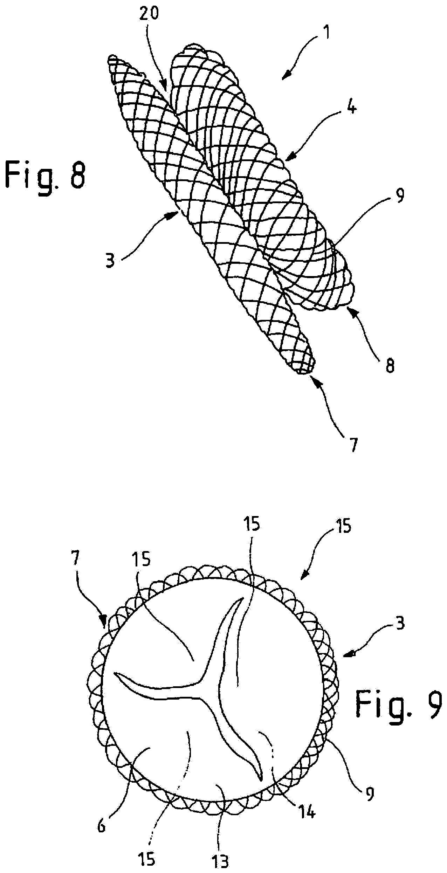

In Figur 8 ist eine perspektivische Ansicht einer vierten Ausführungsform einer erfindungsgemäßen implantierbaren Einrichtung 1 dargestellt. Die implantierbare Einrichtung 1 umfasst einen Grundkörper 2 mit einem ersten Ende 3 und einem zweiten Ende 4, wobei das erste Ende 3 und das zweite Ende 4 jeweils eine Öffnung 5 aufweisen, um eine Fluidverbindung zwischen dem ersten Ende 3 und dem zweiten Ende 4 durch den Grundkörper 2 bereit zu stellen, und ein erstes Membranelement 6 angeordnet innerhalb des Grundkörpers 2, wobei das Membranelement 6 derart ausgebildet ist, dass es die Fluidverbindung durch den Grundkörper 2 in eine erste Flussrichtung freigibt und in eine zweite Flussrichtung, entgegengesetzt zu der ersten Flussrichtung, sperrt. Der Grundkörper 2 weist in einem ersten Betriebszustand (Primärform) ein großes Verhältnis von Länge zu Querausdehnung entlang der Längsachse des Grundkörpers 2 und in einem zweiten Betriebszustand (Sekundärform) ein kleineres Verhältnis von Länge zu Querausdehnung entlang der Längsachse des Grundkörpers 2 auf, wobei der Grundkörper 2 der implantierbaren Einrichtung 1 in Figur 8 in dem zweiten Betriebszustand (Sekundärform) dargestellt ist. Der Grundkörper 2 ist durch Aufbringen einer Kraft gegen elastische Materialkräfte aus der Sekundärform in die Primärform reversibel überführbar.

Der Grundkörper 2 weist in dem zweiten Betriebszustand (Sekundärform) an dem ersten Ende 3 und dem zweiten Ende 4 jeweils ein sich in radialer Richtung des Grundkörpers 2 erstreckenden Verankerungsabschnitt 7, 8 zum Verankern der Einrichtung in einem Organ und/oder Gefäß auf.

Der Grundkörper 2 ist aus einem einzigen drahtartigen Element 9 oder mehreren miteinander drahtartigen Elementen 9 durch verschränkendes Wickeln und/oder Verwinden und/oder Verflechten nach Art eines Gewebes und/oder Geleges und/oder Netzes geformt.

Die zwei sich in radialer Richtung des Grundkörpers 2 erstreckenden Verankerungsabschnitte 7, 8 sind um den Umfang des Grundkörpers 2 am ersten Ende 3 und zweiten Ende 4 des Grundkörpers 2 umlaufend vorgesehen.

Weiterhin umfasst die implantierbare Einrichtung 1 aus Figur 8 wenigstens eine röntgendichte Markierung, insbesondere in Form von Markerplaketten, Markierungen oder Markerdrähten.

Die implantierbare Einrichtung 1 aus Figur 8 besteht ganz oder teilweise aus einem Formgedächtnismaterial, insbesondere Nitinol oder einem Kunststoff mit Formgedächtnis. Insbesondere bei der Verwendung eines Kunststoffs mit Formgedächtnis kann die implantierbare Einrichtung 1 ganz oder teilweise resorbierbar ausgebildet sein.

Dadurch, dass der Grundkörper 2 der implantierbaren Einrichtung 1 gemäß Figur 8 durch Aufbringen einer Kraft gegen elastische Materialkräfte aus der Sekundärform in die Primärform reversibel überführbar ist, ist die implantierbare Einrichtung 1 replazierbar und/oder explantierbar.

Weiterhin ist die implantierbare Einrichtung 1 im zweiten Betriebszustand (Sekundärform) zwischen dem ersten Ende 3 und dem zweiten Ende 4 des Grundkörpers 2 wenigstens teilweise, vorzugsweise vollständig in radialer Richtung deformierbar ausgebildet, so dass sich der Grundkörper 2 an eine Gefäßwandung und/oder den Rand einer Öffnung und/oder den Rand einer defekten Organklappe anlegen kann.

Das einzige drahtartige Element 9 der implantierbaren Einrichtung 1 aus Figur 8 ist aus einem einzelnen Draht 17, einer Drahtlitze 18 aus wenigstens zwei einzelnen Drähten oder einem Mehrfachdraht 19 gebildet. Die beiden Enden des drahtartigen Elements 9 sind am ersten Ende 3 oder zweiten Ende 4 oder in einer Seitenfläche des Grundkörpers 2 angeordnet.

Der erste Verankerungsabschnitt 7 ist an dem ersten Ende 3 des Grundkörpers 2 und der zweite Verankerungsabschnitt 8 an dem zweiten Ende 4 des Grundkörpers 2 angeordnet. Der erste Verankerungsabschnitt 7 und der zweite Verankerungsabschnitt 8 sind in dem zweiten Betriebszustand (Sekundärform) scheibenförmig ausgebildet. Der erste Verankerungsabschnitt 7 ist dabei flach ausgebildet und der zweite Verankerungsabschnitt 8 ist bauchig ausgebildet. Zwischen dem ersten Verankerungsabschnitt 7 und dem zweiten Verankerungsabschnitt 8 weist der Grundkörper 2 der implantierbaren Einrichtung 1 einen Zwischenabschnitt 20 auf, welcher einen geringeren Durchmesser aufweist als der erste Verankerungsabschnitt 7 und der zweite Verankerungsabschnitt 8. Die Durchmesser der zwei Verankerungsabschnitte 7, 8 sind unterschiedlich. Wie der Figur 8 entnommen werden kann, ist der Durchmesser des ersten Verankerungsabschnitts 7 größer als der Durchmesser des zweiten Verankerungsabschnitts 8.

Die zwei Verankerungsabschnitte 7, 8 weisen in dem zweiten Betriebszustand (Sekundärform) des Grundkörpers 2 jeweils einen ersten Teilabschnitt 22 und einen zweiten Teilabschnitt 23 auf, wobei sich jeweils der erste Teilabschnitt 22 in radialer Richtung des Grundkörpers 2 nach außen erstreckt und der zweite Teilabschnitt 23 in radialer Richtung des Grundkörpers 2 nach innen zurückgefaltet ist, insbesondere derart, dass der erste Teilabschnitt 22 und der zweite Teilabschnitt 23 doppellagig aufeinander gefaltet sind. Die bauchige Ausbildung des zweiten Verankerungsabschnitts 8 gemäß Figur 8 bildet dabei ebenfalls einen doppellagig aufeinander gefalteten Verankerungsabschnitt 8 im Sinne der Erfindung.

Das erste Ende 3 und das zweite Ende 4 des Grundkörpers 2 der implantierbaren Einrichtung 1 gemäß Figur 8 weisen jeweils eine oder mehrere verschränkte und/oder nebeneinander angeordnete und/oder ineinander verschlungene Schlingen oder Schlaufen 21 auf. Die Schlingen oder Schlaufen 21 bilden dabei einen gleichmäßigen Rand 28 aus.

Die beiden Enden des drahtartigen Elements 9 sind miteinander geeignet verbindbar oder verbunden, insbesondere durch Auffügen eines weiteren Elements, durch Verdrehen, Verkleben, Schweißen, Löten oder einem anderen Verbindungsverfahren.

Der Querschnitt der implantierbaren Einrichtung 1 aus Figur 8 ist in dem ersten Betriebszustand (Primärform) rund oder oval. In dem zweiten Betriebszustand (Sekundärform) der implantierbaren Einrichtung 1 ist der Querschnitt in den Bereichen, wo kein Verankerungsabschnitt 7, 8 angeordnet ist, rund oder oval. In den Bereichen der Verankerungsabschnitte 7, 8 der implantierbaren Einrichtung 1 aus Figur 8 ist der Durchmesser ebenfalls rund oder oval.

Der Durchmesser der implantierbaren Einrichtung 1 aus Figur 8 ist in dem zweiten Betriebszustand (Sekundärform) in Bereichen außerhalb der Verankerungsabschnitte 7, 8 insbesondere im Bereich des Zwischenabschnitts 20, ungefähr 35 mm. Die Länge der implantierbaren Einrichtung 1 ist in dem zweiten Betriebszustand (Sekundärform) maximal 50 mm.

Der Grundkörper 2 der implantierbaren Einrichtung 1 kann eine oder mehrere Lagen aufweisen.

Figur 9 zeigt eine Draufsicht auf das erste Ende der implantierbaren Einrichtung aus Figur 8. Wie der Figur 9 entnommen werden kann, ist an dem ersten Ende 3 des Grundkörpers 2 der implantierbaren Einrichtung 1 das erste Membranelement 6 angeordnet. Das erste Membranelement 6 besteht aus einem synthetischen oder biologischen Material, beispielsweise aus Polyurethan. Weiterhin ist das erste Membranelement 6 mit einer Beschichtung versehen, zum Schaffen einer Biostabilität, insbesondere einer Titanbeschichtung.

Der Grundkörper 2 der implantierbaren Einrichtung 1 und das erste Membranelement 6 sind lösbar oder unlösbar miteinander verbindbar oder verbunden, insbesondere durch Kleben, Schweißen, Nähen, Aufschmelzen, Tauchen oder einem andern Fügeverfahren.

Das erste Membranelement 6 weist einen Ringabschnitt 13 und einen mit diesem verbundenen Klappenabschnitt 14 auf. Der Klappenabschnitt 14 des ersten Membranelements 6 umfasst gemäß Figur 9 drei Segelelemente 15.

Die in den Figuren 8 und 9 dargestellte vierte Ausführungsform der erfindungsgemäßen implantierbaren Einrichtung 1 mit dem ersten Verankerungsabschnitt 7, dem zweiten Verankerungsabschnitt 8 und dem dazwischen angeordneten Zwischenabschnitt 20 ist insbesondere zum Ersatz einer Viskupidalklappe oder Trikuspidalklappe geeignet. Die implantierbare Einrichtung 1 gemäß der vierten Ausführungsform aus den Figuren 8 und 9 wird derart in den menschlichen oder tierischen Körper 24 angeordnet, dass sich Teile des Papillarmuskels des Herzen im Bereich des Zwischenabschnitts 20 befinden und der erste Verankerungsabschnitt 7 und der zweite Verankerungsabschnitt 8 an unterschiedlichen Seiten des Papillarmuskels an dem Papillarmuskel anliegen, wodurch die implantierbare Einrichtung 1 im Herzen des menschlichen oder tierischen Körpers 24 fixiert wird. Im Falle einer Stenose der natürlichen Klappe sollte die alte natürliche Klappe entfernt werden, insbesondere durch Operation und an dieser Stelle eine implantierbare Einrichtung 1 mit erstem Membranelement 6 als Klappenersatz implantiert werden. Gerade bei starker Verkalkung der natürlichen Herzklappe kann es sich als vorteilhaft erweisen, diese vollständig zu entfernen, da sie dann üblicherweise im Wesentlichen unbeweglich geworden ist. In diesem Fall wäre es ansonsten relativ schwer möglich, die natürliche Klappe an die Gefäßwand zu drücken. Außerdem verbliebe dann eine Verengung in diesem Bereich, die ebenfalls nicht gewünscht ist, da sie eine Strömungsquerschnittsreduzierung und damit eine Druckerhöhung bedeutet und somit gesundheitliche Nachteile für den Patienten mit sich bringt. Im Falle einer Herzklappeninsuffizienz kann die erfindungsgemäß implantierbare Einrichtung 1 auch in der insuffizienten Klappe, beispielsweise Vikuspidalklappe (Mitralklappe) implantiert werden, ohne dass die natürliche Klappe vorher entfernt wird.

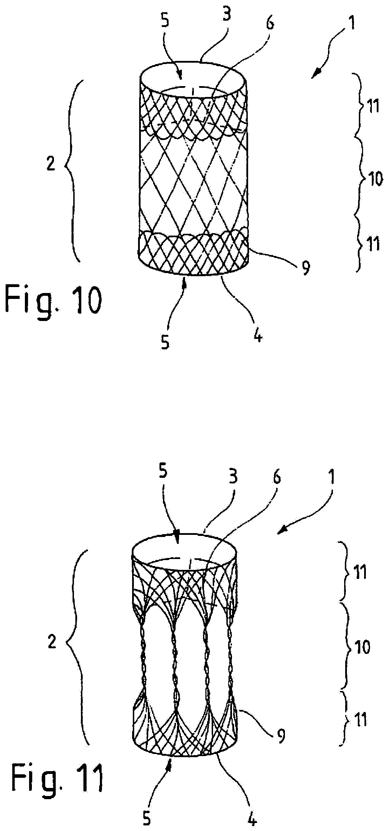

In Figur 10 ist eine perspektivische Ansicht einer fünften Ausführungsform einer erfindungsgemäßen implantierbaren Einrichtung 1 dargestellt. Die implantierbare Einrichtung 1 zur Verwendung im menschlichen oder tierischen Körper 24 zum Ersatz einer Organklappe umfasst einen Grundkörper 2 mit einem ersten Ende 3 und einem zweiten Ende 4, wobei das erste Ende 3 und der zweite Ende 4 jeweils eine Öffnung 5 aufweisen, um eine Fluidverbindung zwischen dem ersten Ende 3 und dem zweiten Ende 4 durch den Grundkörper 2 bereitzustellen und ein erstes Membranelement 6 angeordnet innerhalb oder an einem Ende des Grundkörpers 2, wobei das Membranelement 6 derart ausgebildet ist, dass es die Fluidverbindung durch den Grundkörper 2 in eine erste Flussrichtung freigibt und in eine zweite Flussrichtung, entgegengesetzt zu der ersten Flussrichtung, sperrt. Der Grundkörper 2 weist in einem ersten Betriebszustand (Primärform) ein großes Verhältnis von Länge zu Querausdehnung entlang der Längsachse des Grundkörpers 2 und in einem zweiten Betriebszustand (Sekundärform) ein kleineres Verhältnis von Länge zu Querausdehnung entlang der Längsachse des Grundkörpers 2 auf. Der Grundkörper 2 der implantierbaren Einrichtung 1 ist in Figur 10 in dem ersten Betriebszustand (Primärform) dargestellt.

Der Grundkörper 2 der implantierbaren Einrichtung 1 ist durch Aufbringen einer Kraft gegen elastische Materialkräfte aus der Sekundärform in die Primärform reversibel überführbar.

In dem zweiten Betriebszustand (Sekundärform) weist der Grundkörper 2 an dem ersten Ende 3 und/oder dem zweiten Ende 4 wenigstens einen sich in radialer Richtung des Grundkörpers 2 erstreckenden Verankerungsabschnitt 7, 8 zum Verankern der Einrichtung 1 in einem Organ und/oder einem Gefäß auf.

Der Grundkörper 2 der implantierbaren Einrichtung 1 ist aus einem einzigen drahtartigen Element 9 oder mehreren miteinander verbundenen drahtartigen Elementen 9 durch verschränkendes Wickeln und/oder Verwinden und/oder Verflechten nach Art eines Gewebes und/oder Geleges und/oder Netzes geformt.

Die implantierbare Einrichtung 1 aus Figur 10 weist Bereiche unterschiedlicher Steifigkeit 10, 11 auf. Die Bereiche unterschiedlicher Steifigkeit 10, 11 werden durch unterschiedliches verschränkendes Wickeln und/oder Verwinden und/oder Verflechten erzeugt. In der fünften Ausführungsform der erfindungsgemäßen implantierbaren Einrichtung 1 aus Figur 10 ist ein Bereich geringerer Steifigkeit 10 zwischen Bereichen größerer Steifigkeit 11 angeordnet. Der Bereich geringerer Steifigkeit 10 im Grundkörper 2 ist außerhalb des wenigstens einen Verankerungsabschnitts 7, 8 vorgesehen. Wenn der wenigstens eine Verankerungsabschnitt 7, 8 an dem ersten Ende 3 und/oder zweitem Ende 4 des Grundkörpers 2 angeordnet ist, ist der Bereich geringerer Steifigkeit 10 im Grundkörper 2 folglich im Mittelbereich des Grundkörpers 2 angeordnet.

Der Bereich geringerer Steifigkeit 10 der implantierbaren Einrichtung 1 gemäß der fünften Ausführungsform der erfindungsgemäßen implantierbaren Einrichtung 1 aus Figur 10 ist durch ein unterschiedliches Verflechten des einzigen drahtartigen Elements 9 des Grundkörpers 2 oder mehreren miteinander verbundenen drahtartigen Elementen 9 des Grundkörpers 2 gebildet. Wie der Figur 10 entnommen werden kann, ist die Maschenweite in dem Bereich geringerer Steifigkeit 10 des durch verschränkendes Wickeln und/oder Verwinden und/oder Verflechten erzeugten Gewebes und/oder Geleges und/oder Netzes größer als in den Bereichen größerer Steifigkeit 11.

Alternativ dazu kann der Bereich geringerer Steifigkeit 10 beispielsweise durch ein weiteres verschränkendes Wickeln und/oder Verwinden des einzigen drahtartigen Elements 9 oder mehreren miteinander verbundenen drahtartigen Elementen 9 erzeugt werden, wie beispielsweise in der in Figur 11 dargestellten sechsten Ausführungsform der erfindungsgemäßen implantierbaren Einrichtung 1.

Die implantierbare Einrichtung 1 gemäß der fünften Ausführungsform aus Figur 10 und gemäß der sechsten Ausführungsform aus Figur 11 ist in dem ersten Betriebszustand (Primärform) Stent-förmig ausgebildet. Der Querschnitt der implantierbaren Einrichtung 1 ist in dem ersten Betriebszustand (Primärform) rund oder oval.

Die bezüglich der fünften und sechsten Ausführungsform der implantierbaren Einrichtung 1 beschriebenen Bereiche unterschiedlicher Steifigkeit 10, 11 sind mit den Ausführungsformen zwei bis vier der implantierbaren Einrichtung 1 aus den Figuren 4 bis 9 und mit den nachfolgend beschriebenen Ausführungsformen kombinierbar. Beispielsweise können die implantierbaren Einrichtungen 1 gemäß der fünften und sechsten Ausführungsform aus den Figuren 10 und 11 in den zweiten Betriebszustand (Sekundärform) gemäß einer der Ausführungsformen 2 bis 4 aus den Figuren 4 bis 9 ausgebildet sein.

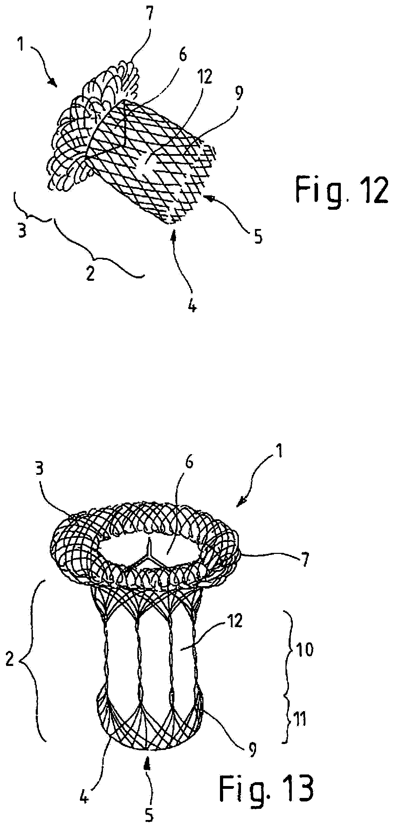

Figur 12 zeigt eine perspektivische Ansicht einer siebten Ausführungsform einer erfindungsgemäßen implantierbaren Einrichtung 1. Die siebte Ausführungsform der erfindungsgemäßen implantierbaren Einrichtung 1 unterscheidet sich von der zweiten Ausführungsform der erfindungsgemäßen implantierbaren Einrichtung 1 dadurch, dass der Grundkörper 2 in dessen Umfangswandung wenigstens eine Öffnung 12 aufweist, um eine Fluidverbindung zwischen dem Inneren des Grundkörpers 2 und einem Gefäß des menschlichen oder tierischen Körpers 24 bereitzustellen. Die wenigstens eine Öffnung 12 weist einen einer Koronararterie entsprechenden Durchmesser auf. In der siebten Ausführungsform gemäß Figur 12 weist der Grundkörper 2 in dessen Umfangswandung zwei Öffnungen 12 auf, welche im implantierten Zustand der implantierbaren Einrichtung 1 derart angeordnet sind, dass sich die zwei Öffnungen 12 mit den Koronararterien überdecken. Die zwei Öffnungen 12 in der Umfangswandung des Grundkörpers 2 sind dabei außerhalb des wenigstens einen Verankerungsabschnitts 7, 8 vorgesehen.

In Figur 13 ist eine perspektivische Ansicht einer achten Ausführungsform einer erfindungsgemäßen implantierbaren Einrichtung 1 dargestellt. Die achte Ausführungsform der erfindungsgemäßen implantierbaren Einrichtung 1 gemäß Figur 13 unterscheidet sich von der siebten Ausführungsform der erfindungsgemäßen implantierbaren Einrichtung 1 gemäß Figur 12 dadurch, dass die wenigstens eine Öffnung in der Umfangswandung des Grundkörpers 2 durch ein weiteres Verwinden, Verflechten oder Wickeln des einzigen drahtartigen Elements 9 des Grundkörpers 2 oder mehreren miteinander verbundenen drahtartigen Elementen 9 des Grundkörpers 2 erzeugt wird, wobei sich durch das weitere Verwinden, Verflechten oder Wickeln des einzigen drahtartigen Elements 9 des Grundkörpers 2 oder mehreren miteinander verbundenen drahtartigen Elementen 9 des Grundkörpers 2 im Bereich der wenigstens einen Öffnung 12 in der Umfangswandung des Grundkörpers 2 eine größere Maschenweite ergibt, als im übrigen Bereich der Umfangswandung des Grundkörpers 2. In der Ausführungsform gemäß Figur 13 ist die wenigstens eine Öffnung 12 in der Umfangswandung des Grundkörpers 2 durch ein weiteres Verwinden des einzigen drahtartigen Elements 9 des Grundkörpers 2 oder mehreren miteinander verbundenen drahtartigen Elementen 9 des Grundkörpers 2 ausgebildet.

Die Ausgestaltungen gemäß der Figuren 12 und 13 sind ebenfalls mit der dritten bis sechsten Ausführungsform der erfindungsgemäßen implantierbaren Einrichtung 1 gemäß der Figuren 6 bis 11 und den nachfolgend beschriebenen Ausführungsformen kombinierbar.

Gemäß einer besonders zweckmäßigen Ausführung ist im Bereich der wenigstens einen Öffnung 12 in der Umfangswandung des Grundkörpers 2 wenigstens eine röntgendichte Markierung angeordnet, insbesondere in Form von Markerplaketten, Markierungen oder Markerdrähten. Dadurch kann während der Implantation der implantierbaren Einrichtung 1 die Positionierung der implantierbaren Einrichtung 1 zu beispielsweise den Koronararterien überprüft werden.

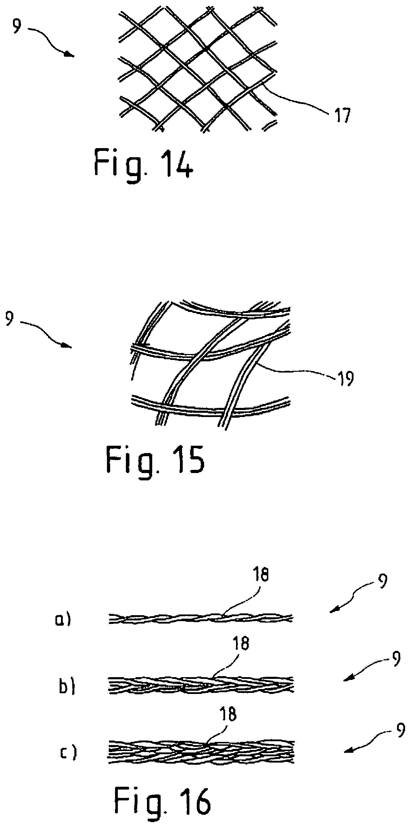

In den Figuren 14 bis 16 sind verschiedenen Ausgestaltungen des einzigen drahtartigen Elements 9 des Grundkörpers 2 der erfindungsgemäßen implantierbaren Einrichtung 1 dargestellt. Mittels des einzigen drahtartigen Elements 9 wird der Grundkörper 2 der implantierbaren Einrichtung 1 durch verschränkendes Wickeln und/oder Verwinden und/oder Verflechten nach Art eines Gewebes und/oder Geleges und/oder Netzes geformt.

Gemäß einer Ausführungsform ist das einzige drahtartige Element 9 aus einem einzelnen Draht 17 gebildet, wie in Figur 14 dargestellt. In Figur 15 ist eine Ausführungsform dargestellt, in welcher das einzige drahtartige Element 9 aus einem Mehrfachdraht 19 gebildet ist. In den Figuren 16 a bis 16 c sind Drahtlitzen 18 dargestellt, die das einzige drahtartige Element 9 der implantierbaren Einrichtung 1 bilden. Die Drahtlitze 18 aus Figur 16 a besteht aus zwei einzelnen Drähten, die Drahtlitze 18 aus Figur 16b ist aus vier einzelnen Drähten gebildet und die Drahtlitze 18 aus Figur 16c aus sechs einzelnen Drähten.

Der Unterschied zwischen einem Mehrfachdraht 19 gemäß Figur 15 und einer Drahtlitze 18 gemäß Figur 16 besteht darin, dass die einzelnen Drähte der Drahtlitze 18 miteinander verdrillt sind, während die einzelnen Drähte eines Mehrfachdrahts parallel zueinander verlaufen.

Zweckmäßigerweise ist ein einzelner Draht des Mehrfachdrahts 19 gemäß Figur 15 oder der Drahtlitze 18 gemäß Figur 16 als Markerdraht ausgebildet, um eine röntgendichte Markierung innerhalb der implantierbaren Einrichtung 1 bereitzustellen.

Die einzelnen Drähte gemäß den Figuren 14 bis 16 bestehen ganz oder teilweise aus einem Formgedächtnismaterial, insbesondere Nitinol oder einem Kunststoff mit Formgedächtnis. Insbesondere bei der Verwendung eines Kunststoffs mit Formgedächtnis kann dieser ganz oder teilweise resorbierbar sein.

Die beiden Enden des drahtartigen Elements 9, wie beispielshaft in den Figuren 14 bis 16 dargestellt, sind an dem ersten Ende 3 oder dem zweiten Ende 4 oder in einer Seitenfläche des Grundkörpers 3 der implantierbaren Einrichtung 1 angeordnet.

Die beiden Enden des drahtartigen Elements 9 sind zweckmäßigerweise miteinander geeignet verbindbar oder verbunden, insbesondere durch Auffügen eines weiteren Elements, durch Verdrehen, Verkleben, Schweißen, Löten oder einem anderen Verbindungsverfahren.

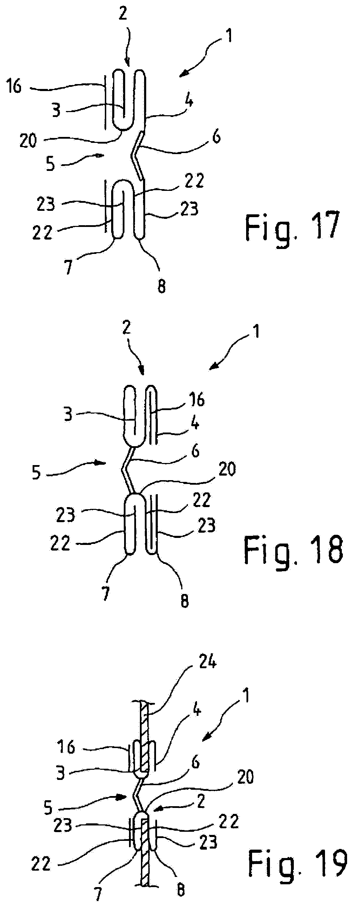

In Figur 17 ist eine Schnittansicht einer neunten Ausführungsform einer erfindungsgemäßen implantierbaren Einrichtung 1 zur Verwendung im menschlichen oder tierischen Körper 24 zum Ersatz einer Organklappe dargestellt. Die implantierbare Einrichtung 1 umfasst einen Grundkörper 2 mit einem ersten Ende 3 und einem zweiten Ende 4, wobei das erste Ende 3 und das zweite Ende 4 jeweils eine Öffnung 5 aufweisen, um eine Fluidverbindung zwischen dem ersten Ende 3 und dem zweiten Ende 4 durch den Grundkörper 2 bereitzustellen, und ein erstes Membranelement 6 angeordnet am zweiten Ende 4 des Grundkörpers 2, wobei das Membranelement 6 derart ausgebildet ist, dass es die Fluidverbindung durch den Grundkörper 2 in eine erste Flussrichtung freigibt und eine zweite Flussrichtung, entgegengesetzt zu der ersten Flussrichtung, sperrt. Der Grundkörper 2 weist in einem ersten Betriebszustand (Primärform) ein großes Verhältnis von Länge zu Querausdehnung entlang der Längsachse des Grundkörpers 2 und in einem zweiten Betriebszustand (Sekundärform) ein kleines Verhältnis von Länge zu Querausdehnung entlang der Längsachse des Grundkörpers 2 auf, wobei die implantierbare Einrichtung 1 in Figur 17 in dem zweiten Betriebszustand (Sekundärform) des Grundkörpers 2 dargestellt ist. Der Grundkörper 2 ist durch Aufbringen einer Kraft gegen elastische Materialkräfte aus der Sekundärform in die Primärform reversibel überführbar.

In dem zweiten Betriebszustand (Sekundärform) weist der Grundkörper 2 an dem ersten Ende 3 und an dem zweiten Ende 4 jeweils einen Verankerungsabschnitt 7, 8 zum Verankern der Einrichtung in einem Organ und/oder einem Gefäß auf. Gemäß der Ausgestaltung aus Figur 17 ist der erste Verankerungsabschnitt 7 an dem ersten Ende 3 des Grundkörpers 2 und der zweite Verankerungsabschnitt 8 an dem zweiten Ende 4 des Grundkörpers 2 angeordnet.

Der Grundkörper 2 ist aus einem einzigen drahtartigen Element 9 oder mehreren miteinander verbundenen drahtartigen Elementen 9 durch verschränkendes Wickeln und/oder Verwinden und/oder Verflechten nach Art eines Gewebes und/oder Geleges und/oder Netzes geformt.

Die sich in radialer Richtung des Grundkörpers 2 erstreckenden Verankerungsabschnitte 7, 8 sind jeweils um den Umfang des Grundkörpers 2 am ersten Ende 3 und zweiten Ende 4 des Grundkörpers 2 umlaufend vorgesehen.

Die implantierbare Einrichtung 1 aus Figur 17 kann Bereich unterschiedlicher Steifigkeit 10, 11 aufweisen, insbesondere wie im Zusammenhang mit den Figuren 10 und 11 beschrieben. Die Bereiche unterschiedlicher Steifigkeit 10, 11 werden beispielsweise durch unterschiedliches verschränkendes Wickeln und/oder Verwinden und/oder Verflechten erzeugt. Zweckmäßigerweise ist ein Bereich geringeren Steifigkeit 10 zwischen Bereichen größerer Steifigkeit 11 angeordnet und der Bereich geringerer Steifigkeit 10 ist im Grundkörper 2 außerhalb der Verankerungsabschnitte 7, 8 vorgesehen.

Wie im Zusammenhang mit den Figuren 12 und 13 beschrieben, kann der Grundkörper 2 in dessen Umfangswandung wenigstens eine Öffnung 12 aufweisen, um eine Fluidverbindung zwischen dem Inneren des Grundkörpers 2 und einem Gefäß des menschlichen oder tierischen Körpers 24 bereitzustellen. Die wenigstens eine Öffnung 12 weist dabei zweckmäßigerweise einen einer Koronararterie entsprechenden Durchmesser auf. Insbesondere weist der Grundkörper 2 in dessen Umfangswandung zwei Öffnungen 12 auf, welche im implantierten Zustand der implantierbaren Einrichtung 1 derart angeordnet sind, dass sich die Öffnungen 12 mit den Koronararterien überdecken. Vorteilhafterweise ist die wenigstens eine Öffnung 12 in der Umfangswandung des Grundkörpers 2 außerhalb der Verankerungsabschnitte 7, 8 vorgesehen.

Die wenigstens eine Öffnung 12 in der Umfangswandung des Grundkörpers 2 wird zum Beispiel durch ein weiteres Verwinden, Verflechten oder Wickeln des einzigen drahtartigen Elements 9 des Grundkörpers 2 oder mehreren miteinander verbundenen drahtartigen Elementen 9 des Grundkörpers 2 erzeugt, wobei sich durch das weitere Verwinden, Verflechten oder Wickeln des einzigen drahtartigen Elements 9 des Grundkörpers 2 oder mehreren miteinander verbundenen drahtartigen Elementen 9 des Grundkörpers 2 im Bereich der wenigstens einen Öffnung 12 in der Umfangswandung des Grundkörpers 2 eine größere Maschenweite ergibt, als im übrigen Bereich der Umfangswandung des Grundkörpers 2.

Wie bereits zu den vorherigen Ausführungsformen ausgeführt, umfasst die implantierbare Einrichtung 1 wenigstens eine röntgendichte Markierung, insbesondere in Form von Markerplaketten, Markierungen oder Markerdrähten. Zweckmäßigerweise ist die wenigstens eine röntgendichte Markierung im Bereich einer Öffnung 12 in der Umfangswandung des Grundkörpers 2 angeordnet, um während des Implantationsvorgangs zu prüfen, ob die Öffnung 12 mit einer Koronararterie in einer Flucht ist.

Die implantierbare Einrichtung 1, insbesondere der Grundkörper 2, gemäß der neunten Ausführungsform aus Figur 17 ist ganz oder teilweise aus einem Formgedächtnismaterial gebildet, insbesondere Nitinol oder einem Kunststoff mit Formgedächtnis. Weiterhin kann die implantierbare Einrichtung 1 ganz oder teilweise aus einem resorbierbaren Material bestehen.

Das erste Membranelement 6 der implantierbaren Einrichtung 1 besteht aus einem synthetischen oder biologischen Material, insbesondere aus Polyurethan. Vorzugsweise ist das erste Membranelement 6 mit einer Beschichtung versehen, zum Schaffen einer Biostabilität, insbesondere einer Titanbeschichtung.

Der Grundkörper 2 und das erste Membranelement 6 sind lösbar oder unlösbar miteinander verbindbar oder verbunden. Insbesondere durch Kleben, Schweißen, Nähen, Aufschmelzen, Tauchen oder einem anderen Fügeverfahren.

Das erste Membranelement 6 kann einen Ringabschnitt 13 und einen mit diesen verbundenen Klappenabschnitt 14 aufweisen, wie beispielsweise bezüglich der zweiten Ausführungsform einer erfindungsgemäßen implantierbaren Einrichtung 1 erläutert und in Figur 5 dargestellt. Der Klappenabschnitt 14 umfasst dabei zweckmäßigerweise drei Segelelemente 15.

In der Ausführungsform gemäß Figur 17 ist das erste Membranelement 6 im zweiten Betriebszustand (Sekundärform) am zweiten Ende 4 des Grundkörpers 2 angeordnet.

Die implantierbare Einrichtung 1 gemäß der neunten Ausführungsform aus Figur 17 umfasst ein zweites Membranelement 16, um die Öffnung 5 am ersten Ende 3 und/oder zweiten Ende 4 teilweise fluiddicht zu verschließen. In der Ausgestaltung gemäß Figur 17 ist das zweite Membranelement 16 im zweiten Betriebszustand (Sekundärform) des Grundkörpers 2 am ersten Ende 3 angeordnet.

Die implantierbare Einrichtung 1 aus Figur 17 ist replazierbar und/oder explantierbar ausgebildet.

Die dargestellte implantierbare Einrichtung 1 ist im zweiten Betriebszustand (Sekundärform) zwischen dem ersten Ende 3 und dem zweiten Ende 4 des Grundkörpers 2 wenigstens teilweise, vorzugsweise vollständig, in radialer Richtung deformierbar ausgebildet, so dass sich der Grundkörper 2 an eine Gefäßwandung und/oder den Rand einer Öffnung und/oder den Rand einer defekten Organklappe anlegen kann. Die implantierbare Einrichtung 1 aus Figur 17 ist somit insbesondere in dem Zwischenabschnitt 20 zwischen dem ersten Verankerungsabschnitt 7 und dem zweiten Verankerungsabschnitt 8 in radialer Richtung deformierbar ausgebildet.

Wie bezüglich der Figuren 14 bis 16 ausgeführt, kann das einzige drahtartige Element 9 aus einem einzelnen Draht 17, einer Drahtlitze 18 aus wenigstens zwei einzelnen Drähten oder einem Mehrfachdraht 19 gebildet werden.

Die beiden Enden des drahtartigen Elements 9 sind zweckmäßigerweise an dem ersten Ende 3 oder dem zweiten Ende 4 oder in einer Seitenfläche des Grundkörpers 2 angeordnet. Zweckmäßigerweise sind die beiden Enden des drahtartigen Elements 9 miteinander geeignet verbindbar oder verbunden, insbesondere durch Auffügen eines weiteren Elements, durch Verdrehen, Verkleben, Schweißen, Löten oder einem anderen Verbindungsverfahren.

Der erste Verankerungsabschnitt 7 und der zweite Verankerungsabschnitt 8 der achten Ausführungsform aus Figur 17 sind in dem zweiten Betriebszustand (Sekundärform) scheibenförmig ausgebildet.

Die Durchmesser der zwei Verankerungsabschnitte 7, 8 sind gleich groß.

Der erste Verankerungsabschnitt 7 und der zweite Verankerungsabschnitt 8 weisen jeweils in dem zweiten Betriebszustand (Sekundärform) des Grundkörpers 2 einen ersten Teilabschnitt 22 und einen zweiten Teilabschnitt 23 auf, wobei sich der erste Teilabschnitt 22 jeweils in radialer Richtung des Grundkörpers 2 nach außen erstreckt und der zweite Teilabschnitt 23 in radialer Richtung des Grundkörpers 2 nach innen zurückgefaltet ist, insbesondere derart, dass der erste Teilabschnitt 22 und der zweite Teilabschnitt 23 doppellagig aufeinander gefaltet sind. Die Zurückfaltung des ersten Verankerungsabschnitts 7 aus Figur 17 erfolgt dabei zur Mitte des Grundköpers 2, während die Zurückfaltung des zweiten Verankerungsabschnitts 8 auf Figur 17 von der Mitte des Grundkörpers 2 weg erfolgt.

Die Materialkonzentration und/oder die Materialstärke innerhalb der implantierbaren Einrichtung 1 kann abschnittsweise unterschiedlich sein, beispielsweise ist die Materialmenge im Randbereich der implantierbaren Einrichtung 1 an die gewünschten mechanischen Eigenschaften angepasst. Insbesondere eine Materialkonzentration 27 im Randbereich der implantierbaren Einrichtung 1 zur partiellen Versteifung.

Das erste Ende 2 und/oder das zweite Ende 4 des Grundkörpers 2 der implantierbaren Einrichtung 1 aus Figur 17 weist eine oder mehrere miteinander verschränkte und/oder nebeneinander angeordnete und/oder ineinander verschlungene Schlingen oder Schlaufen 21 auf. Die Schlingen oder Schlaufen 21 können einen gleichmäßigen Rand 28 oder einen ungleichmäßigen Rand 29 ausbilden. Der ungleichmäßige Rand 29 wird beispielsweise durch Schlingen oder Schlaufen 21 unterschiedlicher Größe ausgebildet, zum Beispiel durch Schlingen oder Schlaufen 21 mit zwei unterschiedlichen Größen. Zweckmäßigerweise ist die Anordnung der unterschiedlich großen Schlingen oder Schlaufen 21 gleichmäßig, beispielsweise auch eine große Schlinge oder Schlaufe 21 nach drei kleinen Schlingen oder Schlaufen 21.

Der Grundkörper 2 der implantierbaren Einrichtung 1 ist in dem ersten Betriebszustand (Primärform) Stent-förmig ausgebildet. Der Querschnitt der implantierbaren Einrichtung 1 ist in dem ersten Betriebszustand (Primärform) zweckmäßigerweise rund oder oval. In dem zweiten Betriebszustand (Sekundärform) der implantierbaren Einrichtung 1 ist der Querschnitt in den Bereichen, wo kein Verankerungsabschnitt 7, 8 angeordnet ist, rund oder oval, zweckmäßigerweise ebenfalls in den Bereichen, wo ein Verankerungsabschnitt 7, 8 angeordnet ist.

Beispielsweise ist der Durchmesser der implantierbaren Einrichtung 1 in dem zweiten Betriebszustand (Sekundärform) in den Bereichen außerhalb der Verankerungsabschnitte 7,8 ungefähr 35 mm und die Länge der implantierbaren Einrichtung ist in dem zweiten Betriebszustand (Sekundärform) maximal 50 mm.

Der Grundkörper 2 der implantierbaren Einrichtung 1 kann eine oder mehrere Lagen aufweisen.

Die in einer Schnittansicht in Figur 18 dargestellte zehnte Ausführungsform einer erfindungsgemäßen implantierbaren Einrichtung 1 unterscheidet sich von der neunten Ausführungsform der erfindungsgemäßen implantierbaren Einrichtung 1 aus Figur 17 dadurch, dass das erste Membranelement 6 innerhalb des Grundkörpers 2 zwischen dem ersten Ende 3 und dem zweiten Ende 4 des Grundkörpers 2 angeordnet ist, vorzugsweise ungefähr mittig in Längsrichtung des Grundkörpers 2 zwischen dem ersten Ende 3 und dem zweiten Ende 4 des Grundkörpers 2. Weiterhin ist in der zehnten Ausführungsform gemäß Figur 18 das zweite Membranelement 16 am zweiten Ende 4 der implantierbaren Einrichtung 1 angeordnet, zwischen dem ersten Teilabschnitt 22 und dem zweiten Teilabschnitt 23 des zweiten Verankerungsabschnitt 8.

Figur 19 zeigt eine Schnittansicht einer elften Ausführungsform einer erfindungsgemäßen implantierbaren Einrichtung 1 in einem im menschlichen oder tierischen Körper 24 implantierten Zustand. Die elfte Ausführungsform gemäß Figur 19 unterscheidet sich von der zehnten Ausführungsform gemäß Figur 18 dadurch, dass das zweite Membranelement 16 am ersten Ende 3 der implantierbaren Einrichtung 1 angeordnet ist. Das zweite Membranelement 16 und das erste Membranelement 6 sind in dem zweiten Betriebszustand (Sekundärform) der implantierbaren Einrichtung 1 benachbart zueinander angeordnet, wodurch insbesondere in die zweite Flussrichtung eine fluiddichte Verbindung erzielt wird.

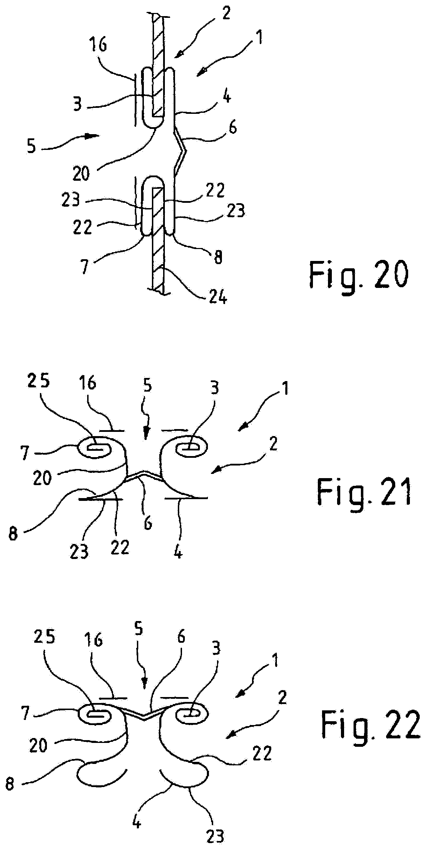

Eine zwölfte Ausführungsform einer erfindungsgemäßen implantierbaren Einrichtung 1 im implantierten Zustand ist in Figur 20 dargestellt. Die zwölfte Ausführungsform aus Figur 20 unterscheidet sich von der neunten Ausführungsform aus Figur 17 dadurch, dass das erste Membranelement 6 derart angeordnet ist, dass die erste Flussrichtung und die zweite Flussrichtung der zwölften Ausführungsform entgegengesetzt ausgebildet sind zu der achten Ausführungsform aus Figur 17.

In Figur 21 ist eine dreizehnte Ausführungsform einer nicht erfindungsgemäßen implantierbaren Einrichtung 1 zur Verwendung im menschlichen oder tierischen Körper 24 zum Ersatz einer Organklappe in einer Schnittansicht dargestellt. Die implantierbare Einrichtung 1 umfasst einen Grundkörper 2 mit einem ersten Ende 3 und einem zweiten Ende 4, wobei das erste Ende 3 und das zweite Ende 4 jeweils eine Öffnung 5 aufweisen, um eine Fluidverbindung zwischen dem ersten Ende 3 und dem zweiten Ende 4 durch den Grundkörper 2 bereitzustellen, und ein erstes Membranelement 6 angeordnet innerhalb des Grundkörpers 2, wobei das Membranelement 6 derart ausgebildet ist, dass es die Fluidverbindung durch den Grundkörper 2 in eine erste Flussrichtung freigibt und in eine zweite Flussrichtung, entgegengesetzt zu der ersten Flussrichtung, sperrt. Der Grundkörper 2 weist in einem ersten Betriebszustand (Primärform) ein großes Verhältnis von Länge zu Querausdehnung entlang der Längsachse des Grundkörpers 2 und in einem zweiten Betriebszustand (Sekundärform) ein kleineres Verhältnis von Länge zu Querausdehnung entlang der Längsachse des Grundkörpers 2 auf. Der Grundkörper 2 ist durch Aufbringen einer Kraft gegen elastische Materialkräfte aus der Sekundärform in die Primärform reversibel überführbar. In Figur 18 ist die implantierbare Einrichtung 1 in dem zweiten Betriebszustand (Sekundärform) dargestellt.

In dem dargestellten zweiten Betriebszustand (Sekundärform) weist der Grundkörper 2 an dem ersten Ende 3 und dem zweiten Ende 4 jeweils einen sich in radialer Richtung des Grundkörpers 2 erstreckenden Verankerungsabschnitt 7, 8 zum Verankern der Einrichtung 1 in einem Organ und/oder einem Gefäß auf.

Der Grundkörper 2 der implantierbaren Einrichtung 1 ist aus einem einzigen drahtartigen Element 9 oder mehreren miteinander verbundenen drahtartigen Elementen 9 durch verschränkendes Wickeln und/oder Verwinden und/oder Verflechten nach Art eines Gewebes und/oder Geleges und/oder Netzes geformt.

Die sich in radialer Richtung des Grundkörpers 2 erstreckenden Verankerungsabschnitte 7, 8 sind um den Umfang des Grundkörpers 2 am ersten Ende 3 und zweiten Ende 4 des Grundkörpers 2 umlaufend vorgesehen.

Analog zu der fünften und sechsten Ausführungsform aus den Figuren 10 und 11 kann die dreizehnte Ausführungsform gemäß Figur 21 ebenfalls Bereiche unterschiedlicher Steifigkeit 10, 11 aufweisen.

Der Grundkörper 2 der implantierbaren Einrichtung 1 aus Figur 21 kann in dessen Umfangswandung wenigstens eines Öffnung 12 aufweisen, um eine Fluidverbindung zwischen dem Inneren des Grundkörpers 2 und einem Gefäß des menschlichen oder tierischen Körpers 24 bereitzustellen, wie insbesondere zu den Ausführungsformen aus den Figuren 12 und 13 beschrieben.

Zweckmäßigerweise besteht die implantierbare Einrichtung 1 aus Figur 21 ganz oder teilweise aus einem Formgedächtnismaterial, insbesondere Nitinol oder einem Kunststoff mit Formgedächtnis und umfasst weiterhin wenigstens eine röntgendichte Markierung, insbesondere in Form von Markerplaketten, Markierungen oder Markerdrähten. Die implantierbare Einrichtung 1 kann ebenfalls ganz oder teilweise aus einem resorbierbaren Material bestehen.

Das erste Membranelement 6 der implantierbaren Einrichtung 1 aus Figur 21 besteht aus einem synthetischen oder biologischen Material und ist zweckmäßigerweise mit einer Beschichtung versehen, zum Schaffen einer Biostabilität, insbesondere einer Titanbeschichtung.

Der Grundkörper 2 und das erste Membranelement 6 der implantierbaren Einrichtung 1 sind lösbar oder unlösbar miteinander verbindbar oder verbunden, insbesondere durch Kleben, Schweißen, Nähen, Aufschmelzen, Tauchen oder einem anderen Fügeverfahren. Das erste Membranelement 6 ist im zweiten Betriebszustand (Sekundärform) zwischen dem ersten Ende 3 und dem zweiten Ende 4 des Grundkörpers 2 angeordnet, vorzugsweise mittig in Längsrichtung des Grundkörpers 2 zwischen dem ersten Ende 3 und dem zweiten Ende 4 des Grundkörpers 2.

Der Grundkörper 2 der implantierbaren Einrichtung 1 weist im zweiten Betriebszustand (Sekundärform) am ersten Ende 3 ein zweites Membranelement 16 auf, um die Öffnung 5 am ersten Ende 3 teilweise fluiddicht zu verschließen.

Die implantierbare Einrichtung 1 nach Figur 21 ist replazierbar und explantierbar ausgebildet.

Weiterhin ist die implantierbare Einrichtung 1 aus Figur 21 im zweiten Betriebszustand (Sekundärform) zwischen dem ersten Ende 3 und dem zweiten Ende 4 des Grundkörpers 2, also insbesondere im Bereich des Zwischenabschnitts 20, wenigstens teilweise, vorzugsweise vollständig in radialer Richtung deformierbar ausgebildet, so dass sich der Grundkörper 2 an eine Gefäßwandung und/oder den Rand einer Öffnung und/oder den Rand einer defekten Organklappe anlegen kann.

Das einzige drahtartige Element 9 der implantierbaren Einrichtung 1 ist aus einem einzelnen Draht 17, einer Drahtlitze 18 aus wenigstens zwei einzelnen Drähten oder einem Mehrfachdraht 19 gebildet, wie insbesondere zu den in den Figuren 14 bis 16 beschriebenen Ausführungsformen offenbart.

Die beiden Enden des drahtartigen Elements 9 sind am ersten Ende 3 oder zweiten Ende 4 oder in einer Seitenfläche des Grundkörpers 2 angeordnet. Weiterhin sind die beiden Enden des drahtartigen Elements 9 miteinander geeignet verbindbar oder verbunden, insbesondere Auffügen eines weiteren Elements, durch Verdrehen, Verkleben, Schweißen, Löten oder einem anderen Verbindungsverfahren.

Der erste Verankerungsabschnitt 7 der implantierbaren Einrichtung 1 ist in dem zweiten Betriebszustand (Sekundärform) an dem ersten Ende 3 des Grundkörpers 2 und der zweite Verankerungsabschnitt 8 an dem zweiten Ende 4 des Grundkörpers 2 angeordnet. Der zweite Verankerungsabschnitt 8 ist in dem zweiten Betriebszustand (Sekundärform) schirmförmig ausgebildet, wie in Figur 21 dargestellt. Der zweite Verankerungsabschnitt 8 weist in dem zweiten Betriebszustand (Sekundärform) des Grundkörpers 2 einen ersten Teilabschnitt 22 und einen zweiten Teilabschnitt 23 auf, wobei sich der erste Teilabschnitt 22 in radialer Richtung des Grundkörpers 2 nach außen erstreckt und der zweite Teilabschnitt 23 in radialer Richtung des Grundkörpers 2 nach innen zurückgefaltet ist, insbesondere derart, dass der erste Teilabschnitt 22 und der zweite Teilabschnitt 23 doppellagig aufeinander gefaltet sind.

Wie in Figur 21 dargestellt, ist der erste Verankerungsabschnitt 7 in dem zweiten Betriebszustand (Sekundärform) des Grundkörpers 2 aufgewickelt, vorzugsweise schneckenförmig. Die Aufwicklung des ersten Verankerungsabschnitts 7 ist zur Mitte des Grundkörpers 2 hin angeordnet.

Wie bereits zuvor bezüglich der vorherigen Ausführungsformen beschrieben, kann die Materialkonzentration 27 und/oder die Materialstärke innerhalb der implantierbaren Einrichtung 1 abschnittsweise unterschiedlich sein. Insbesondere kann die Materialmenge im Randbereich der implantierbaren Einrichtung 1 an die gewünschten mechanischen Eigenschaften angepasst sein, insbesondere kann eine Materialkonzentration 27 im Randbereich der implantierbaren Einrichtung 1 zur partiellen Versteifung vorgesehen sein.

Das erste Ende 3 und/oder das zweite Ende 4 des Grundkörpers 2 der implantierbaren Einrichtung 1 weist vorzugsweise eine oder mehrere miteinander verschränkte und/oder nebeneinander angeordnete und/oder ineinander verschlungene Schlingen oder Schlaufen 21 auf. Die Schlingen oder Schlaufen 21 bilden einen gleichmäßigen Rand 28 oder ungleichmäßigen Rand 29 aus. Ein ungleichmäßiger Rand 29 wird durch Schlingen oder Schlaufen 21 unterschiedlicher Größe ausgebildet, beispielsweise durch Schlingen oder Schlaufen 21 mit zwei unterschiedlichen Größen. Die Anordnung der unterschiedlich großen Schlingen oder Schlaufen 21 kann gleichmäßig sein, beispielsweise eine große Schlinge oder Schlaufe 21 nach drei kleinen Schlingen oder Schlaufen 21.

In dem nicht dargestellten ersten Betriebszustand (Primärform) ist der Grundkörper 2 der implantierbaren Einrichtung 1 aus Figur 21 Stent-förmig ausgebildet und weist einen runden oder ovalen Querschnitt auf.

In dem zweiten Betriebszustand (Sekundärform) weist die implantierbare Einrichtung 1 aus Figur 21 ebenfalls in den Bereichen, wo kein Verankerungsabschnitt 7, 8 angeordnet ist, einen runden oder ovalen Querschnitt auf.

Die dargestellte implantierbare Einrichtung 1 weist in dem zweiten Betriebszustand (Sekundärform) in den Bereichen außerhalb der Verankerungsabschnitte 7, 8 einen Durchmesser von ungefähr 35 mm auf und ist maximal 50 mm lang.

Der Grundkörper 2 der implantierbaren Einrichtung 1 aus Figur 21 kann eine oder mehrere Lagen aufweisen.

Figur 22 zeigt eine Schnittansicht einer vierzehnten Ausführungsform einer nicht erfindungsgemäßen implantierbaren Einrichtung 1 zur Verwendung im menschlichen oder tierischen Körper 24 zum Ersatz einer Organklappe. Die vierzehnte Ausführungsform gemäß Figur 22 unterscheidet sich von der dreizehnten Ausführungsform gemäß Figur 21 zum Einen dadurch, dass das erste Membranelement 6 in dem zweiten Betriebszustand (Sekundärform) des Grundkörpers 2 am ersten Ende 3 der implantierbaren Einrichtung 1 angeordnet ist. Zum Anderen umfasst die implantierbare Einrichtung 1 gemäß Figur 22 ein zweites Membranelement 16, welches in dem zweiten Betriebszustand (Sekundärform) an dem ersten Ende 3 der implantierbaren Einrichtung 1 angeordnet ist. Das erste Membranelement 6 und das zweite Membranelement 16 sind am ersten Ende 3 des Grundkörpers 2 in dem zweiten Betriebszustand (Sekundärform) benachbart zueinander angeordnet. Dadurch wird insbesondere die Fluiddichtigkeit der implantierbaren Einrichtung 1 am ersten Ende 3 verbessert.

Weiterhin unterscheidet sich sie vierzehnte Ausführungsform gemäß Figur 22 von der dreizehnten Ausführungsform aus Figur 21 dadurch, dass der zweite Verankerungsabschnitt 8 bauchig ausgebildet ist. Dadurch wird erreicht, dass der zweite Verankerungsabschnitt 8 deformierbar ausgebildet ist und sich beispielsweise besser an eine Gefäßwandung oder der Innenwandung einer Herzkammer anlegen kann.

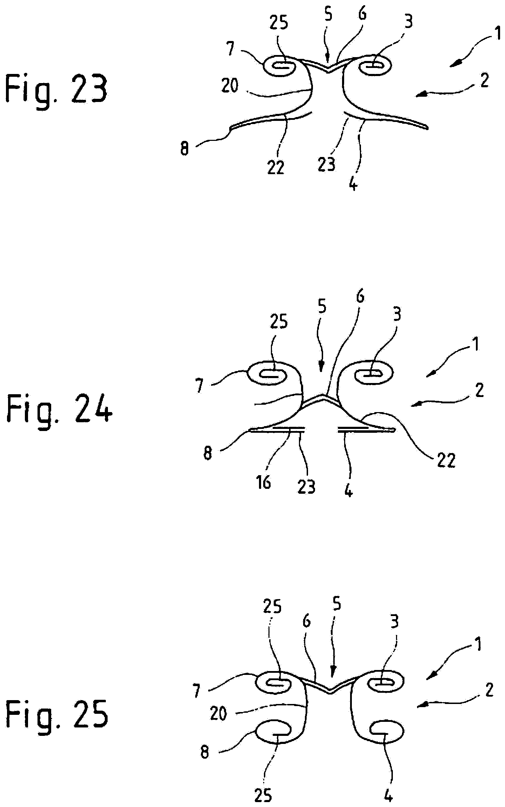

In Figur 23 ist eine Schnittansicht einer fünfzehnten Ausführungsform einer implantierbaren Einrichtung 1 zur Verwendung im menschlichen oder tierischen Körper 24 zum Ersatz einer Organklappe dargestellt. Die fünfzehnte Ausführungsform gemäß Figur 23 unterscheidet sich von der vierzehnten Ausführungsform gemäß Figur 22 dadurch, dass kein zweites Membranelement 16 an dem ersten Ende 3 der implantierbaren Einrichtung 1 angeordnet ist. Weiterhin ist der zweite Verankerungsabschnitt 8 an dem zweiten Ende 4 des Grundkörpers 2 in dem zweiten Betriebszustand (Sekundärform) gekrümmt ausgebildet. Die Krümmung des zweiten Verankerungsabschnitts 8 ist dabei von der Mitte des Grundkörpers 2 in der Sekundärform weg gerichtet, so dass der Scheitelpunkt der Krümmung in Richtung des Zwischenabschnitts 20 des Grundkörpers 2 angeordnet ist.

Weiterhin ist der Durchmesser des zweiten Verankerungsabschnitts 8 größer als der Durchmesser des ersten Verankerungsabschnitts 7.

Eine sechszehnte Ausführungsform einer nicht erfindungsgemäßen implantierbaren Einrichtung 1 zur Verwendung im menschlichen oder tierischen Körper 24 zum Ersatz einer Organklappe ist in einer Schnittansicht in Figur 24 dargestellt. Die sechszehnte Ausführungsform gemäß Figur 24 unterscheidet sich durch das innerhalb der Zurückfaltung des zweiten Verankerungsabschnitts 8 angeordnete zweite Membranelement 16 von der dreizehnten Ausführungsform gemäß Figur 21. Durch das zweite Membranelement 16 innerhalb der Zurückfaltung des zweiten Verankerungsabschnitts 8 wird die Fluiddichtigkeit im Bereich des zweiten Endes 4 des Grundkörpers 2 erhöht.

Figur 25 zeigt eine Schnittansicht einer siebzehnten Ausführungsform einer nicht erfindungsgemäßen implantierbaren Einrichtung 1 zur Verwendung im menschlichen oder tierischen Körper 24 zum Ersatz einer Organklappe. Die siebzehnte Ausführungsform gemäß Figur 25 unterscheidet sich von der fünfzehnten Ausführungsform gemäß Figur 23 dadurch, dass der zweite Verankerungsabschnitt 8 ebenfalls durch eine Aufwicklung 25 des Grundkörpers 3 ausgebildet ist.

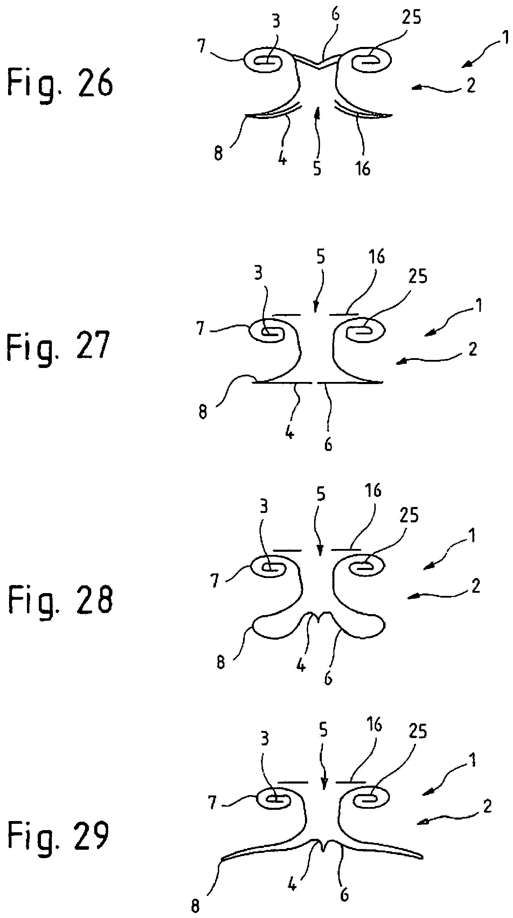

Die in Figur 26 dargestellte Schnittansicht einer achtzehnte Ausführungsform einer nicht erfindungsgemäßen implantierbaren Einrichtung 1 zur Verwendung im menschlichen oder tierischen Körper 24 zum Ersatz einer Organklappe unterscheidet sich von der fünfzehnten Ausführungsform einer nicht erfindungsgemäßen implantierbaren Einrichtung 1 aus Figur 23 durch die Krümmung des zweiten Verankerungsabschnitts 8, den innerhalb der Zurückfaltung des zweiten Verankerungsabschnitts 8 angeordneten zweiten Membranelements 16 und der Größe des zweiten Verankerungsabschnitts 8. Die Krümmung des zweiten Verankerungsabschnitts 8 der implantierbaren Einrichtung 1 gemäß Figur 26 ist zur Mitte des Grundkörpers 2 gerichtet.

Figur 27 zeigt eine Schnittansicht mit einer neunzehnten Ausführungsform einer nicht erfindungsgemäßen implantierbaren Einrichtung 1 zur Verwendung im menschlichen oder tierischen Körper 24 zum Ersatz einer Organklappe. Die neunzehnte Ausführungsform gemäß Figur 27 unterscheidet sich von der dreizehnten Ausführungsform gemäß Figur 21 dadurch, dass das erste Membranelement 6 an dem zweiten Ende 4 des Grundkörpers 2 angeordnet ist und einstückig mit dem Grundkörper 2 ausgebildet ist.

Die in Figur 28 dargestellt Schnittansicht einer zwanzigsten Ausführungsform einer nicht erfindungsgemäßen implantierbaren Einrichtung 1 zur Verwendung im menschlichen oder tierischen Körper 24 zum Ersatz einer Organklappe unterscheidet sich von der neunzehnten Ausführungsform einer nicht erfindungsgemäßen implantierbaren Einrichtung 1 gemäß Figur 27 durch die bauchige Ausgestaltung des zweiten Verankerungsabschnitts 8.

Die einundzwanzigste Ausführungsform einer nicht erfindungsgemäßen implantierbaren Einrichtung 1 zur Verwendung im menschlichen oder tierischen Körper 24 zum Ersatz einer Organklappe, welche in Figur 29 in einer Schnittansicht dargestellt ist, unterscheidet sich von der neunzehnten Ausführungsform einer nicht erfindungsgemäßen implantierbaren Einrichtung 1 gemäß Figur 27 durch die gekrümmte Ausgestaltung des zweiten Verankerungsabschnitts 8, wobei die Krümmung des zweiten Verankerungsabschnitts 8 von der Mitte des Grundkörpers 2 weg gerichtet ist. Weiterhin ist der Durchmesser des zweiten Verankerungsabschnitts 8 größer als der Durchmesser des ersten Verankerungsabschnitts 7.

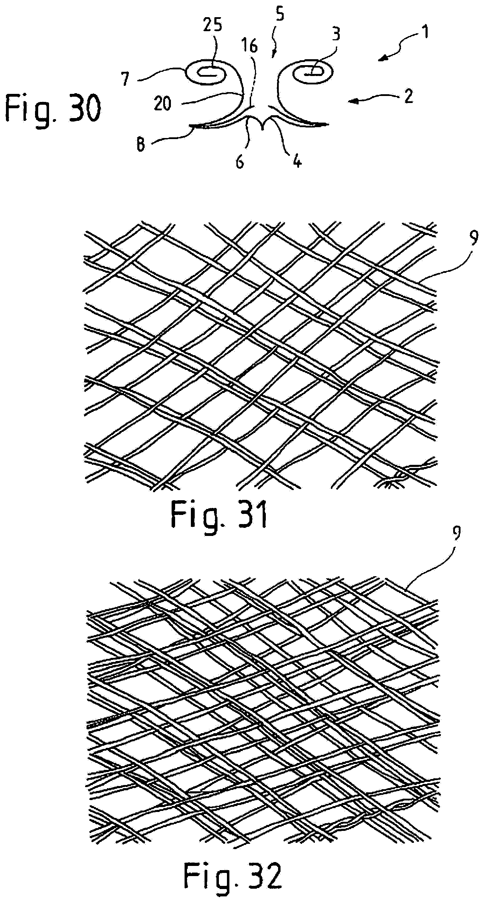

In Figur 30 ist eine Schnittansicht einer zweiundzwanzigsten Ausführungsform einer nicht erfindungsgemäßen implantierbaren Einrichtung 1 zur Verwendung im menschlichen oder tierischen Körper 24 zum Ersatz einer Organklappe dargestellt. Im Gegensatz zu der einundzwanzigsten Ausführungsform der nicht erfindungsgemäßen implantierbaren Einrichtung 1 aus Figur 29 erfolgt die Krümmung des zweiten Verankerungsabschnitts 8 zur Mitte des Grundkörpers 2. Weiterhin ist innerhalb des doppellagig ausgebildeten zweiten Verankerungsabschnitts 8 ein zweites Membranelement 16 angeordnet.

Sämtliche zuvor beschriebenen Ausführungsformen der implantierbaren Einrichtung 1 zur Verwendung im menschlichen oder tierischen Körper 24 zum Ersatz einer Organklappe können eine oder mehrere Lagen aufweisen. In Figur 31 ist eine Detailansicht eines Grundkörpers 2 einer erfindungsgemäßen oder nicht erfindungsgemäßen implantierbaren Einrichtung 1 mit zwei Lagen dargestellt. Figur 32 zeigt eine Detailansicht eines Grundkörpers 2 einer erfindungsgemäßen oder nicht erfindungsgemäßen implantierbaren Einrichtung 1 mit drei Lagen.

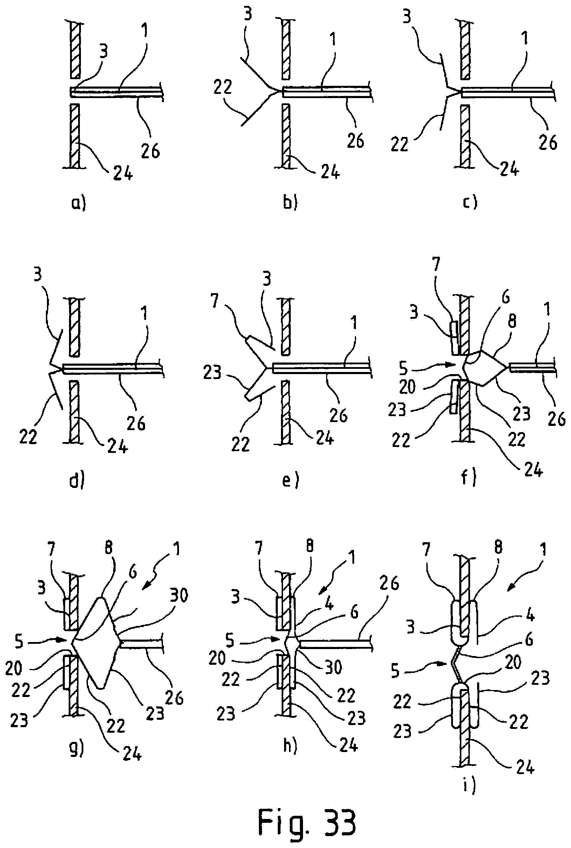

In Figur 33 ist ein beispielhafter Implantationsvorgang einer erfindungsgemäßen implantierbaren Einrichtung 1 zur Verwendung im menschlichen oder tierischen Körper 24 zum Ersatz einer Organklappe dargestellt. Die implantierbare Einrichtung 1 ist in dem ersten Betriebszustand (Primärform) Stent-förmig ausgebildet und innerhalb eines Katheters 26 angeordnet. Der Katheter 26 wird beispielsweise über das Venensystem des menschlichen oder tierischen Körpers 24 in die Nähe des Implantationsorts geführt. In Figur 33 a ist dargestellt, wie das Ende des Katheters 26 innerhalb einer Öffnung in den menschlichen oder tierischen Körper 24 angeordnet ist, in welcher die implantierbare Einrichtung 1 implantiert werden soll.

Nachfolgend wird die implantierbare Einrichtung 1 aus dem Katheter 26 hinausgeschoben. Da die implantierbare Einrichtung 1 aus einem Formgedächtnismaterial besteht und durch Aufbringen einer Kraft gegen elastische Materialkräfte aus der Sekundärform in die Primärform reversibel überführt worden ist, nimmt die implantierbare Einrichtung 1 nach dem Austreten aus dem Katheter 26 die Sekundärform an. In Figur 33b ist dargestellt, wie das erste Ende 3 der implantierbaren Einrichtung 1 aus dem Katheter 26 austritt. Das erste Ende 3 der implantierbaren Einrichtung 1, welches zuerst aus dem Katheter 26 austritt, bildet den ersten Teilabschnitt 22 des ersten Verankerungsabschnitts 7.

In Figur 33c ist dargestellt, wie der erste Teilabschnitt 22 des ersten Verankerungsabschnitts 7 vollständig aus dem Katheter ausgetreten ist und sich ungefähr rechtwinklig von dem Katheter 26 weg erstreckt, in radialer Richtung der implantierbaren Einrichtung 1.

Bei einem weiteren Hinausschieben der implantierbaren Einrichtung 1 aus dem Katheter 26 bildet sich die Zurückfaltung des ersten Teilabschnitts 22 des ersten Verankerungsabschnitts 7 aus und der zweite Teilabschnitt 23 des ersten Verankerungsabschnitts 7 tritt nachfolgend aus dem Katheter 26 aus, wie in den Figuren 33d und e dargestellt.

Sobald sich der erste Verankerungsabschnitt 7 vollständig ausgebildet hat, wird der Katheter von der Implantationsstelle solange entfernt, bis sich der erste Verankerungsabschnitt 7 an die Oberfläche des menschlichen oder tierischen Körpers 24 anlegt, insbesondere der erste Teilabschnitt 22 des ersten Verankerungsabschnitts 7. Der erste Teilabschnitt 22 und der zweite Teilabschnitt 23 bilden zusammen den ersten Verankerungsabschnitt 7 mit einer Zurückfaltung, so dass der erste Verankerungsabschnitt 7 doppellagig abgebildet ist. Nachfolgend wird die erfindungsgemäße implantierbare Einrichtung 1 weiter aus dem Katheter 26 hinausgeschoben, wie in Figur 33f dargestellt. Weiterhin ist in Figur 33f zu sehen, dass in dem Zwischenabschnitt 20 zwischen dem ersten Verankerungsabschnitt 7 und dem zweiten Verankerungsabschnitt 8 ein erstes Membranelement 6 angeordnet ist, wobei das Membranelement 6 derart ausgebildet ist, dass es die Fluidverbindung durch den Grundkörper 2 in eine erste Flussrichtung freigibt und in eine zweite Flussrichtung, entgegengesetzt der ersten Flussrichtung, sperrt. Der zweite Verankerungsabschnitt 8 der implantierbaren Einrichtung 1 umfasst ebenfalls einen ersten Teilabschnitt 22 und einen zweiten Teilabschnitt 23.

Beim weiteren Austreten der implantierbaren Einrichtung 1 aus dem Katheter 26 nimmt der zweite Verankerungsabschnitt 8 die Sekundärform an, wobei der erste Teilabschnitt 22 und der zweite Teilabschnitt 23 des zweiten Verankerungsabschnitts 8 ebenfalls doppellagig aufeinander gefaltet werden. Die Zurückfaltung des ersten Verankerungsabschnitts 7 ist dabei in Richtung der Öffnung innerhalb des menschlichen oder tierischen Körpers 24 angeordnet und die Zurückfaltung des zweiten Verankerungsabschnitts 8 ist von der Öffnung innerhalb des menschlichen oder tierischen Körpers 24 weg gerichtet. Figur 33 g zeigt eine implantierbare Einrichtung 1 während der Implantation mit einem vollständig ausgebildeten ersten Verankerungsabschnitt 7 und einem teilweise ausgebildeten zweiten Verankerungsabschnitt 8.

In Figur 33h ist eine implantierbare Einrichtung 1 mit vollständig ausgebildeten ersten und zweiten Verankerungsabschnitten 7, 8 dargestellt. Die Implantation der implantierbaren Einrichtung 1 erfolgt beispielsweise mittels Führungsdrähten 30, die durch den Katheter 26 mit der implantierbaren Einrichtung 1 verbunden sind. In Figur 33h ist dargestellt, wie die Führungsdrähte 30 noch mit dem zweiten Ende 4 des Grundkörpers 2 der implantierbaren Einrichtung 1 verbunden sind. Durch ein Zurückziehen der Führungsdrähte 30 in den Katheter 26 lässt sich die implantierbare Einrichtung 1 in den Katheter 26 zurückziehen und replazieren bzw. explantieren.

Wenn die implantierbare Einrichtung 1 wie gewünscht innerhalb der Öffnung des menschlichen oder tierischen Körpers 24 platziert wurde, werden die Führungsdrähte 30 von der implantierbaren Einrichtung 1 gelöst und die implantierbare Einrichtung 1 verbleibt in dem menschlichen oder tierischen Körper 24. Die erfindungsgemäße implantierbare Einrichtung 1 ersetzt nun beispielsweise eine defekte Organklappe in dem menschlichen oder tierischen Körper 24.

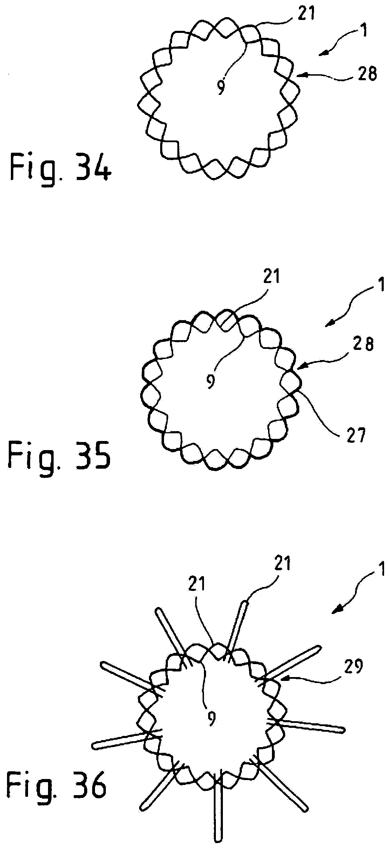

In Figur 34 ist eine Detailansicht einer ersten Ausgestaltung eines Randbereichs einer implantierbaren Einrichtung 1 dargestellt. Der Randbereich der implantierbaren Einrichtung, also das erste Ende 3 oder das zweite Ende 4 des Grundkörpers 2 wird durch mehrere miteinander verschränkte und/oder nebeneinander angeordnete und/oder ineinander verschlungene Schlingen oder Schlaufen 21 gebildet. In Figur 34 bilden die Schlingen oder Schlaufen 21 einen gleichmäßigen Rand 28 aus.

In Figur 35 ist eine Detailansicht einer zweiten Ausgestaltung eines Randbereichs einer erfindungsgemäßen oder nicht erfindungsgemäßen implantierbaren Einrichtung 1 dargestellt. Die Ausgestaltung gemäß Figur 35 unterscheidet sich von der Ausgestaltung gemäß Figur 34 dadurch, dass die implantierbare Einrichtung 1 abschnittsweise unterschiedliche Materialkonzentrationen und/oder Materialstärken aufweist. Gemäß Figur 35 ist im Randbereich der implantierbaren Einrichtung 1 eine Materialkonzentration 27 zur partiellen Versteifung vorgesehen.

Figur 36 zeigt eine Detailansicht einer dritten Ausgestaltung eines Randbereichs einer erfindungsgemäßen oder nicht erfindungsgemäßen implantierbaren Einrichtung 1. Die Schlingen oder Schlaufen 21 der implantierbaren Einrichtung 1 gemäß Figur 36 bilden einen ungleichmäßigen Rand 29 aus. Der ungleichmäßige Rand 29 wird durch Schlingen oder Schlaufen 21 mit zwei unterschiedlichen Größen ausgebildet. Die Anordnung der unterschiedlich großen Schlingen oder Schlaufen 21 ist in Figur 36 gleichmäßig, nämlich eine große Schlinge oder Schlaufe 21 nach zwei kleinen Schlingen oder Schlaufen 21. Die Ausgestaltung nach Figur 36 ist insbesondere bei einer Zurückfaltung des Randbereichs vorteilhaft, da die vorstehenden großen Schlingen oder Schlaufen 21 bei der Zurückfaltung des Randbereichs beispielsweise in den Papillarmuskels des Herzens eingreifen können und somit die implantierbare Einrichtung 1 ortsfest verankern.

### Bezugszeichenliste

- 1: implantierbare Einrichtung
- 2: Grundkörper
- 3: erstes Ende
- 4: zweites Ende
- 5: Öffnung
- 6: erstes Membranelement
- 7: erster Verankerungsabschnitt
- 8: zweiter Verankerungsabschnitt
- 9: drahtartiges Element
- 10: Bereich geringerer Steifigkeit
- 11: Bereich größerer Steifigkeit
- 12: Öffnung Umfangswandung
- 13: Ringabschnitt
- 14: Klappenabschnitt
- 15: Segelelement
- 16: zweites Membranelement
- 17: einzelner Draht
- 18: Drahtlitze
- 19: Mehrfachdraht
- 20: Zwischenabschnitt
- 21: Schlingen/Schlaufen
- 22: erster Teilabschnitt
- 23: zweiter Teilabschnitt
- 24: menschlicher oder tierischer Körper
- 25: Aufwicklung
- 26: Katheter
- 27: Materialkonzentration
- 28: gleichmäßiger Rand
- 29: ungleichmäßiger Rand
- 30: Führungsdraht

## Patentansprüche

1. Implantierbare Einrichtung (1) zur Verwendung im menschlichen oder tierischen Körper (24) zum Ersatz einer Organklappe umfassend:
einen Grundkörper (2) mit einem ersten Ende (3) und einem zweiten Ende (4), wobei das erste Ende (3) und das zweite Ende (4) jeweils eine Öffnung (5) aufweisen, um eine Fluidverbindung zwischen dem ersten Ende (3) und dem zweiten Ende (4) durch den Grundkörper (2) bereitzustellen; und
ein erstes Membranelement (6) angeordnet innerhalb oder an einem Ende (3, 4) des Grundkörpers (2), wobei das Membranelement (6) derart ausgebildet ist, dass es die Fluidverbindung durch den Grundkörper (2) in eine erste Flussrichtung freigibt und in eine zweite Flussrichtung, entgegengesetzt zu der ersten Flussrichtung, sperrt;
wobei der Grundkörper (2) in einem ersten Betriebszustand, einer Primärform, ein großes Verhältnis von Länge zu Querausdehnung entlang der Längsachse des Grundkörpers (2) und in einem zweiten Betriebszustand, einer Sekundärform, ein kleineres Verhältnis von Länge zu Querausdehnung entlang der Längsachse des Grundkörpers (2) aufweist; und
wobei der Grundkörper (2) durch Aufbringen einer Kraft gegen elastische Materialkräfte aus der Sekundärform in die Primärform reversibel überführbar ist; und
wobei der Grundkörper (2) aus einem einzigen drahtartigen Element (9) oder mehreren miteinander verbundenen drahtartigen Elementen (9) durch verschränkendes Wickeln und/oder Verwinden und/oder Verflechten nach Art eines Gewebes und/oder Geleges und/oder Netzes geformt ist,
wobei der Grundkörper (2) in dem zweiten Betriebszustand, der Sekundärform, an dem ersten Ende (3) und/oder dem zweiten Ende (4) wenigstens einen sich in radialer Richtung des Grundkörpers (2) erstreckenden Verankerungsabschnitt (7, 8) zum Verankern der Einrichtung (1) in einem Organ und/oder einem Gefäß aufweist,
**dadurch gekennzeichnet, dass**
der wenigstens eine Verankerungsabschnitt (7, 8) in dem zweiten Betriebszustand, der Sekundärform, des Grundkörpers (2) einen ersten Teilabschnitt (22) und einen zweiten Teilabschnitt (23) aufweist, wobei sich der erste Teilabschnitt (22) in radialer Richtung des Grundkörpers (2) nach außen erstreckt und der zweite Teilabschnitt (23) in radialer Richtung des Grundkörpers (2) nach innen zurückgefaltet ist, derart, dass der erste Teilabschnitt (22) und der zweite Teilabschnitt (23) doppellagig aufeinander gefaltet sind,
wobei die Zurückfaltung des wenigstens einen Verankerungsabschnitts (6, 7) in Richtung der Mitte des Grundkörpers (2) angeordnet ist.

2. Implantierbare Einrichtung (1) nach Anspruch 1,
**dadurch gekennzeichnet, dass**
das erste Membranelement (6) aus einem synthetischen oder biologischen Material besteht, insbesondere aus Polyurethan, wobei
das erste Membranelement (6) vorzugsweise mit einer Beschichtung versehen ist, zum Schaffen einer Biostabilität, insbesondere einer Titanbeschichtung.

3. Implantierbare Einrichtung (1) nach einem der Ansprüche 1 bis 2,
**dadurch gekennzeichnet, dass**
der Grundkörper (2) und das erste Membranelement (6) lösbar oder unlösbar miteinander verbindbar oder verbunden sind, insbesondere durch Kleben, Schweißen, Nähen, Aufschmelzen, Tauchen oder einem anderen Fügeverfahren.

4. Implantierbare Einrichtung (1) nach einem der Ansprüche 1 bis 3,
**dadurch gekennzeichnet, dass**
das erste Membranelement (6) einen Ringabschnitt (13) und/oder einen mit diesem verbundenen Klappenabschnitt (14) aufweist, wobei
der Klappenabschnitt (14) vorzugsweise drei Segelelemente (15) umfasst.

5. Implantierbare Einrichtung (1) nach einem der Ansprüche 1 bis 4,
**dadurch gekennzeichnet, dass**
das erste Membranelement (6) im zweiten Betriebszustand, der Sekundärform, am ersten Ende (3) des Grundkörpers (2), am zweiten Ende (4) des Grundkörpers (2) oder zwischen dem ersten Ende (3) und dem zweiten Ende (4) des Grundkörpers (2) angeordnet ist, vorzugsweise mittig in Längsrichtung des Grundkörpers (2) zwischen dem ersten Ende (3) und dem zweiten Ende (4) des Grundkörpers (2).

6. Implantierbare Einrichtung (1) nach einem der Ansprüche 1 bis 5,
**dadurch gekennzeichnet, dass**
die implantierbare Einrichtung (1) im zweiten Betriebszustand der Sekundärform, zwischen dem ersten Ende (3) und dem zweiten Ende (4) des Grundkörpers (2) wenigstens teilweise, vorzugsweise vollständig in radialer Richtung deformierbar ausgebildet ist, so dass sich der Grundkörper (2) an eine Gefäßwandung und/oder den Rand einer Öffnung und/oder den Rand einer defekten Organklappe anlegen kann.

7. Implantierbare Einrichtung (1) nach einem der Ansprüche 1 bis 6,
**dadurch gekennzeichnet, dass**
die mehreren miteinander verbundenen drahtartigen Elemente (9) aus einem einzelnen Draht (17), einer Drahtlitze (18) aus wenigstens zwei einzelnen Drähten und/oder einem Mehrfachdraht (19) ausgebildet sind, insbesondere, dass die implantierbare Einrichtung (1) in Bereichen mit unterschiedlicher Steifigkeit (10, 11) aus unterschiedlichen drahtartigen Elementen (9), insbesondere einem einzelnen Draht (17), einer Drahtlitze (18) aus wenigstens zwei einzelnen Drähten und/oder einem Mehrfachdraht (19) ausgebildet ist.

8. Implantierbare Einrichtung (1) nach einem der Ansprüche 1 bis 7,
**dadurch gekennzeichnet, dass**
die Zurückfaltung oder Aufwicklung des wenigstens einen Verankerungsabschnitts (7, 8) zur Mitte des Grundkörpers (2) hin oder von der Mitte des Grundkörpers (2) weg angeordnet ist.

9. Implantierbare Einrichtung (1) nach einem der Ansprüche 1 bis 8,
**dadurch gekennzeichnet, dass**
das erste Ende (3) und/oder das zweite Ende (4) des Grundkörpers (2) eine oder mehrere miteinander verschränkte und/oder nebeneinander angeordnete und/oder ineinander verschlungene Schlingen oder Schlaufen (21) aufweist.

10. Implantierbare Einrichtung (1) nach Anspruch 9,
**dadurch gekennzeichnet, dass**
die Schlingen oder Schlaufen (21) einen ungleichmäßigen Rand (29) ausbilden, wobei
der ungleichmäßige Rand (29) durch Schlingen oder Schlaufen (21) unterschiedlicher Größe ausgebildet wird.

11. Implantierbare Einrichtung (1) nach Anspruch 10,
**dadurch gekennzeichnet, dass**
der ungleichmäßige Rand (29) durch Schlingen oder Schlaufen (21) mit zwei unterschiedlichen Größen ausgebildet wird, wobei
die Anordnung der unterschiedlich großen Schlingen oder Schlaufen (21) gleichmäßig ist, beispielsweise eine große Schlinge oder Schlaufe (21) nach drei kleinen Schlingen oder Schlaufen (21).

12. Implantierbare Einrichtung (1) nach einem der Ansprüche 1 bis 11,
**dadurch gekennzeichnet, dass**
der Grundkörper (2) eine oder mehrere Lagen aufweist.

13. Implantierbare Einrichtung (1) nach einem der Ansprüche 1 bis 12,
**dadurch gekennzeichnet, dass**
der Grundkörper (2) und das erste Membranelement (6) einstückig ausgebildet sind.

## Claims

1. Implantable device (1) for use in the human or animal body (24) to replace an organ valve, comprising:
a main body (2) having a first end (3) and a second end (4), wherein the first end (3) and the second end (4) each have an opening (5) to provide a fluid connection through the main body (5) between first end (3) and the second end (4); and
a first membrane element (6) arranged inside or at one end (3, 4) of the main body (2), wherein the membrane element (6) is formed in such a manner that it allows a fluid connection through the main body (2) in a first flow direction and blocks the same in a second flow direction opposite the first flow direction;
wherein the main body (2) has a large ratio of length to transverse expansion along the longitudinal axis of the main body (2) in a first operating state, a primary form, and a smaller ration of length to transverse expansion along the longitudinal axis of the main body (2) in a second operating state, s secondary form; and
wherein the main body (2) can be reversibly transferred from the secondary form to the primary form counter to elastic material forces by the application of a force; and
wherein the main body (2) is formed from a single wire-like element (9) or from a plurality of wire-like elements (9) connected to each other by means of interlocking winding and/or twisting and/or weaving in the manner of a woven and/or layered fabric and/or net;
wherein the main body (2) in the second operating state, the secondary form, has at the first end (3) and/or second end (4) at least one anchoring member (7, 8) extending in a radial direction of the main body (2) for anchoring the device (1) in a organ and/or a vessel,
**characterized in that**
the at least one anchoring member (7, 8) in the second operating state, the secondary form, of the main body (2) has a first sub-portion (22) and a second sub-portion (23), wherein the first sub-portion (22) extends in a radial direction of the main body (2) outwardly and the second sub-portion (23) is folded backwards in a radial direction of the main body (2) inwardly, in such a way, that the first sub-portion (22) and the second sub-portion (23) are folded onto another to a double-layer,
wherein the backfolding of the at least one anchoring member (7, 8) is directed towards the middle of the main body (2).

2. Implantable device (1) according to claim 1,
**characterized in that**
the first membrane element (6) consist of a synthetic or biological material, particularly of polyurethane,
wherein the first membrane element (6) preferably has a coating for establishing a biostability, particularly a titanium coating.

3. Implantable device (1) according to one of claims1 to 2,
**characterized in that**
the main body (2) and the first membrane element (6) are detachable or inseparable connected or connectable to each other, particularly by gluing, welding, sewing, fusing, dip-coating or another joining technology.

4. Implantable device (1) according to one of claims 1 to 3,
**characterized in that**
the first membrane element (6) has a ring portion (13) and/or a valve portion (14) connected thereto,
wherein the valve portion (14) preferably comprises three leaflet elements (15).

5. Implantable device (1) according to one of claims 1 to 4,
**characterized in that**
the first membrane element (6) is located in the second operating state, the secondary form, at the first end (3) of the main body (2), at the second end (4) of the main body (2) or between the first end (3) and the second end (4) of the main body (2), preferably centrally between the first end (3) and the second end (4) of the main body (2) along the longitudinal axis of the main body (2)

6. Implantable device (1) according to one of claims 1 to 5,
**characterized in that**
the implantable device (1) is in the second operating state, the secondary form, between the first end (3) and the second end (4) of the main body (2) at least partially, preferably completely, deformable in a radial direction, such that the main body (2) can adapt to a vessel wall and/or circumference of an opening and/or edge of a defect organ valve.

7. Implantable device (1) according to one of claims 1 to 6,
**characterized in that**
the multiple to each other connected wire-like elements (9) consist of a single wire (17), a wire strand (18) of at least two single wires and/or a multiple wire (19), particularly that the implantable device (1) in areas with different rigidity (10, 11) consists of different wire-like elements (9), particularly of a single wire (17), a wire strand (18) of at least two single wires and/or a multiple wire (19).

8. Implantable device (1) according to one of claims 1 to 7,
**characterized in that**
wherein the backfolding or coiling of the at least one anchoring member (7, 8) is directed towards the middle of the main body (2) or away from the middle of the main body (2).

9. Implantable device (1) according to one of claims 1 to 8,
**characterized in that**
the first end (3) and/or second end (4) of the main body (2) has one or multiple slings or loops (21) interlaced with each other and/or located adjacent to each other and/or intertwined with each other.

10. Implantable device (1) according to claim 9,
**characterized in that**
the slings or loops (21) form an irregular rim (29),
wherein the irregular rim (29) is formed by slings or loops (21) of different sizes.

11. Implantable device (1) according to claim 10,
**characterized in that**
the irregular rim (29) is formed by slings or loops (21) of two different sizes,
wherein the arrangement of slings or loops (21) with different sizes is regular, for example one big sling or loop (21) after three small slings or loops (21).

12. Implantable device (1) according to one of claims 1 to 1,
**characterized in that**
the main body (2) has one or multiple layers.

13. Implantable device (1) according to one of claims 1 to 12,
**characterized in that**
the main body (2) and the first membrane element (6) are built integrally.

## Revendications

1. Dispositif implantable (1) destiné à une utilisation dans le corps humain ou animal (24) pour remplacer un clapet organique, comprenant :
un corps principal (2) ayant une première extrémité (3) et une seconde extrémité (4), dans lequel la première extrémité (3) et la seconde extrémité (4) ont chacune une ouverture (5) pour fournir une connexion fluidique à travers le corps principal (5) entre une première extrémité (3) et la seconde extrémité (4) ; et
un premier élément de membrane (6) disposé à l'intérieur ou au niveau d'une extrémité (3, 4) du corps principal (2), dans lequel l'élément de membrane (6) est formé de manière à ce qu'il permet une connexion fluidique à travers le corps principal (2) dans une première direction de flux, et bloque celle-ci dans une seconde direction de flux opposée à la première direction de flux ;
dans lequel le corps principal (2) présente un rapport de longueur important sur une expansion transversale le long de l'axe longitudinal du corps principal (2) dans un premier état de fonctionnement, une forme primaire, et un rapport de longueur inférieur sur une expansion transversale le long de l'axe longitudinal du corps principal (2) dans un second état de fonctionnement, une forme secondaire ; et
dans lequel le corps principal (2) peut être transféré de façon réversible de la forme secondaire à la forme primaire à l'encontre de forces de matériaux élastiques par l'application d'une force ; et
dans lequel le corps principal (2) est formé d'un seul élément filaire (9) ou à partir d'une pluralité d'éléments filaires (9) reliés les uns aux autres par un enroulement et/ou une torsion et/ou un tissage imbriqués à la manière d'un tissu et/ou d'un filet tissé et/ou stratifié ;
dans lequel le corps principal (2) dans le second état de fonctionnement, la forme secondaire, présente au niveau d'une première extrémité (3) et/ou d'une seconde extrémité (4) au moins un élément d'ancrage (7, 8) s'étendant dans une direction radiale du corps principal (2) pour ancrer le dispositif (1) dans un organe et/ou un vaisseau,
**caractérisé en ce que**
l'au moins un élément d'ancrage (7, 8) dans le second état de fonctionnement, la forme secondaire, du corps principal (2) présente une première sous-partie (22) et une seconde sous-partie (23), dans lequel la première sous-partie (22) s'étend dans une direction radiale du corps principal (2) vers l'extérieur et la seconde sous-partie (23) est rabattue vers l'arrière dans une direction radiale du corps principal (2) vers l'intérieur, de sorte que la première sous-partie (22) et la seconde sous-partie (23) sont rabattues l'une sur l'autre en une double couche,
dans lequel le repliage de l'au moins un élément d'ancrage (7, 8) se dirige vers le milieu du corps principal (2).

2. Dispositif implantable (1) selon la revendication 1,
**caractérisé en ce que**
le premier élément de membrane (6) se compose d'un matériau synthétique ou biologique, notamment du polyuréthane,
dans lequel le premier élément de membrane (6) possède de préférence un revêtement pour établir une bio stabilité, notamment un revêtement en titane.

3. Dispositif implantable (1) selon l'une des revendications 1 à 2,
**caractérisé en ce que**
le corps principal (2) et le premier élément de membrane (6) sont détachables ou inséparables, reliés ou pouvant être reliés l'un à l'autre, notamment par collage, soudage, couture, fusion, revêtement par immersion ou une autre technologie d'assemblage.

4. Dispositif implantable (1) selon l'une des revendications 1 à 3,
**caractérisé en ce que**
le premier élément de membrane (6) possède une portion annulaire (13) et/ou une portion de clapet (14) qui y est connectée,
dans lequel la portion de clapet (14) comprend notamment trois éléments de valve (15).

5. Dispositif implantable (1) selon l'une des revendications 1 à 4,
**caractérisé en ce que**
le premier élément de membrane (6) est situé dans le second état de fonctionnement, la forme secondaire, au niveau de la première extrémité (3) du corps principal (2), au niveau de la seconde extrémité (4) du corps principal (2) ou entre la première extrémité (3) et la seconde extrémité (4) du corps principal (2), de préférence de manière centrale entre la première extrémité (3) et la seconde extrémité (4) du corps principal (2) le long de l'axe longitudinal du corps principal (2).

6. Dispositif implantable (1) selon l'une des revendications 1 à 5,
**caractérisé en ce que**
le dispositif implantable (1) se trouve dans le second état de fonctionnement, la forme secondaire, entre la première extrémité (3) et la seconde extrémité (4) du corps principal (2) au moins partiellement, de préférence complètement, déformable dans une direction radiale, de sorte que le corps principal (2) peut s'adapter à une paroi de vaisseau et/ou une circonférence d'une ouverture et/ou d'un bord d'un clapet organique défectueux.

7. Dispositif implantable (1) selon l'une des revendications 1 à 6,
**caractérisé en ce que**
le multiple de chacun des autres éléments filaires connectés (9) se compose d'un seul fil (17), d'un toron de fils (18) d'au moins deux fils uniques et/ou de fils multiples (19), notamment **en ce que** le dispositif implantable (1) dans des zones présentant une rigidité différente (10, 11) se compose de différents éléments filaires (9), en particulier d'un seul fil (17), d'un toron de fils (18) d'au moins deux fils uniques et/ou de fils multiples (19).

8. Dispositif implantable (1) selon l'une des revendications 1 à 7,
**caractérisé en ce que**
le repliage ou enroulement de l'au moins un élément d'ancrage (7, 8) se dirige vers le milieu du corps principal (2) ou à distance du milieu du corps principal (2).

9. Dispositif implantable (1) selon l'une des revendications 1 à 8,
**caractérisé en ce que**
la première extrémité (3) et/ou la seconde extrémité (4) du corps principal (2) présentent une ou de multiples élingues ou boucles (21) entrelacées les unes avec les autres et/ou situées de manière adjacente les unes par rapport aux autres et/ou entremêlées les unes avec les autres.

10. Dispositif implantable (1) selon la revendication 9,
**caractérisé en ce que**
les élingues ou les boucles (21) forment un rebord irrégulier (29),
dans lequel le rebord irrégulier (29) est formé par des élingues ou des boucles (21) de différentes tailles.

11. Dispositif implantable (1) selon la revendication 10,
**caractérisé en ce que**
le rebord irrégulier (29) est formé par des élingues ou des boucles (21) de deux tailles différentes,
dans lequel l'agencement des élingues ou des boucles (21) de tailles différentes est régulier, par exemple une grosse élingue ou boucle (21) après trois petites élingues ou boucles (21).

12. Dispositif implantable (1) selon l'une des revendications 1 à 11,
**caractérisé en ce que**
le corps principal (2) présente une ou de multiples couches.

13. Dispositif implantable (1) selon l'une des revendications 1 à 12,
**caractérisé en ce que**
le corps principal (2) et le premier élément de membrane (6) sont construits d'une seule pièce.
